(19) Europäisches Patentamt
European Patent Office
Office européen des brevets

(11) EP 0 702 517 B1

(12) EUROPEAN PATENT SPECIFICATION

(45) Date of publication and mention of the grant of the patent:
**15.09.2004 Bulletin 2004/38**

(21) Application number: **94920194.1**

(22) Date of filing: **09.06.1994**

(51) Int Cl.[7]: **A01N 31/14**, A01N 33/12, A01N 37/02, A01N 37/06, A01N 37/10, A01N 43/16, B27K 3/00, B27K 3/34, B27K 3/36, B27K 3/38, C07C 211/63, A01N 33/02, A01N 33/04

(86) International application number:
**PCT/US1994/006699**

(87) International publication number:
**WO 1994/028715 (22.12.1994 Gazette 1994/28)**

# (54) QUATERNARY AMMONIUM AND WATERPROOFING/PRESERVATIVE COMPOSITIONS

QUARTÄRES AMMONIUM UND WASSERDICHTUNG-/KONSERVIERUNGSZUSAMMENSETZUNG

AMMONIUM QUATERNAIRE ET COMPOSITIONS IMPERMEABILISANTES/ANTIPUTRIDES

(84) Designated Contracting States:
**AT BE CH DE DK ES FR GB GR IE IT LI LU MC NL PT SE**
Designated Extension States:
**SI**

(30) Priority: **09.06.1993 US 74136**
**09.06.1993 US 74312**
**09.06.1993 US 74313**
**09.06.1993 US 74314**

(43) Date of publication of application:
**27.03.1996 Bulletin 1996/13**

(60) Divisional application:
**01100855.4 / 1 114 704**
**01101120.2 / 1 122 044**
**01101121.0 / 1 121 857**

(73) Proprietor: **LONZA INC.**
**Fair Lawn New Jersey 07410 (US)**

(72) Inventor: **WALKER, Leigh**
**Macungie, PA 18062 (US)**

(74) Representative: **Riegler, Norbert Hermann**
**Lonza AG**
**Patentabteilung**
**Postfach**
**4002 Basel (CH)**

(56) References cited:

| | |
|---|---|
| **EP-A- 0 291 074** | **EP-A- 0 293 192** |
| **EP-A- 0 293 556** | **EP-A- 0 340 816** |
| **EP-A- 0 472 973** | **EP-A- 0 542 199** |
| **DE-B- 2 916 304** | **FR-A- 1 518 427** |
| **JP-A- 59 039 337** | **US-A- 2 295 504** |
| **US-A- 3 169 983** | **US-A- 3 281 458** |
| **US-A- 3 366 672** | **US-A- 3 666 690** |
| **US-A- 4 129 645** | **US-A- 4 205 063** |
| **US-A- 4 216 168** | **US-A- 4 510 074** |
| **US-A- 4 532 161** | **US-A- 4 545 855** |
| **US-A- 4 585 795** | **US-A- 4 732 817** |
| **US-A- 4 929 454** | **US-A- 4 950 329** |
| **US-A- 5 001 156** | **US-A- 5 045 570** |
| **US-A- 5 169 624** | **US-A- 5 334 763** |
| **US-A- 5 354 434** | |

- **DATABASE WPI Section Ch, Week 9113 Derwent Publications Ltd., London, GB; Class A60, AN 91-092262 XP002064210 & JP 03 038552 A (TERAGUCHI SANGYO KK)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- DATABASE WPI Section Ch, Week 8939 Derwent Publications Ltd., London, GB; Class C03, AN 89-281351 XP002064211 & JP 01 204701 A (KAO CORP)
- DATABASE WPI Section Ch, Week 9122 Derwent Publications Ltd., London, GB; Class B05, AN 91-159342 XP002064212 & JP 03 093752 A (TOYO GOSEI KOGYO KK)
- DATABASE WPI Section Ch, Week 8902 Derwent Publications Ltd., London, GB; Class A97, AN 89-013289 XP002084863 & JP 63 290703 A (SANYO MOKUZAI BOFU KK) , 28 November 1988
- DATABASE WPI Section Ch, Week 8618 Derwent Publications Ltd., London, GB; Class A60, AN 86-116594 XP002084864 & JP 61 057305 A (SANYO MOKUZAIBOFU) , 24 March 1986
- DATABASE WPI Section Ch, Week 8939 Derwent Publications Ltd., London, GB; Class C02, AN 89-280839 XP002084865 & JP 01 203304 A (KAO CORP) , 16 August 1989
- E. MÜLLER (ED.): "Methoden der organischen Chemie (Houben-Weyl), Vol. XI/2. Stickstoffverbindungen II und III." 1958 , GEORG THIEME VERLAG , STUTTGART XP002084862 * page 620, paragraph VII - page 626, paragraph 1 *
- WHITTEN et al., "General Chemistry", published 1981, by SAUNDERS COLLEGE PUBLISHING (PHILADELPHIA), see page 334.
- "Fine and Functional Chemicals", Catalogue 91-16, published 1991, by AKZO, see pages 1 and 3-20.
- PROCEEDINGS OF THE AMERICAN WOOD-PRESERVERS' ASSOCIATION, Vol. 83, issued 1987, PRESTON et al., "Recent Research on Alkylammonium Compounds in the U.S.", see pages 331-348.
- JOURNAL OF PHYSICAL CHEMISTRY, Volume 93, issued 1989, D.D. MILLER et al., "Fluorescence Quenching in Double-Chained Surfactants. 1. Theory of Quenching in Micelles and Vesicles", pages 323-333.
- JOURNAL OF THE AMERICAN CHEMICAL SOCIETY, Volume 106, issued 1984, J. BRADY et al., "Spontaneous Vesicles", pages 4279-80.
- CHEMICAL ABSTRACTS, Vol. 100, issued 19 March 1984, S. TAKEMOTO et al., "Development of a New Cosmetic O/W, Emulsion System Stabilized with Insoluble Complexes", see Abstract No. 91129; & J. SCCJ. 17(1), 52-9.

## Description

FIELD OF THE INVENTION

This invention relates to wood preservation compositions. Polyhydroxyl or polyether hydroxyl esters of fatty acids and polyether hydroxides in combination with quaternary ammonium compositions and a solvent are useful as waterproofing wood preservation compositions. Preferred quaternary ammonium compositions include $C_8$-$C_{12}$ alkyl quaternary ammonium hydroxides and carbonates.

## BACKGROUND OF THE INVENTION

Quaternary ammonium compounds (quats), and particularly didecyldimethylammonium chloride (DDAC)

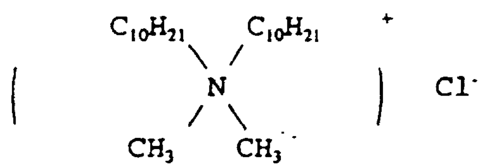

(I)

are commonly used as wood preservatives because they possess resistance properties to fungi and termites, to loss of strength, and to electrical sensitivity similar to those of commonly used acidic copper/chromium/arsenic solution (CCA) or ammoniacal copper and arsenic salt solution preservatives. See Proc of the Am. Wood Pres. Assoc., 80: 191-210 (1984). Although chloride quats do not include potentially dangerous heavy metals, didecyldimethylammonium chloride leaches rapidly in soil (Nicholas et al., Forest Prod. J., 41:41 (1991), and therefore, does require coupling with copper salt.

Findlay et al., U.S. Patent No. 4,929,454, disclose a method,of preserving wood by impregnation with a quaternary ammonium compound and at least one of zinc and copper, wherein the quat anion is chosen from the group consisting of hydroxide, chloride, bromide, nitrate, bisulfate, acetate, bicarbonate, and carbonate, formate, borate and fatty acids. These quats have distinct environmental and safety advantages over commonly used acidic copper/chromium/arsenic solution (CCA) or ammoniacal copper and arsenic salt solution preservatives in that potentially dangerous heavy metals are not included. The Findlay et al. quats require copper or zinc in order to render .them relatively insoluble and to prevent them from leaching out of a treated substrate. The use of copper or zinc in the above formulations may yet raise environmental and corrosion concerns.

Additionally, didecyldimethylammonium chloride tends to absorb preferentially to the surface of the wood and does not uniformly treat the whole substrate. Finally, DDAC treated wood shows a surface erosion or ages upon exposure to light. See Preston et al., Proc. Am. Wood Pres. Assoc., 83:331 (1987).

The biocidal activities of various chloride quats against bacteria, fungi, and algae are tabulated in Cationic Surfactants, E. Jungerman Ed., pp. 56-57, Marcel Dekker, Inc., 1969. Nicholas, "Interaction of Preservatives with Wood," Chemistry of Solid Wood, Advances in Chemistry Series #207, Powell ed., (A.C.S. 1984), notes that didecyldimethylammonium compounds and particularly DDAC are potential biocides. Preston, J.A.O.C.S. 60:567 (1983), concurs and suggests that' maximum fungitoxicity is exhibited with dialkyldimethyl compounds having $C_{10}$-$C_{12}$ alkyl groups. Butcher et al., Chem Abstracts No. 91:152627b, suggest that the presence of an acid or a base can affect the activity of didecyldimethylammonium quats.

Didecyldimethylammonium acetate was used as a phase transfer catalyst for an oxidation in Chem Abstracts No. 97:9175. A wood preservative was prepared by autoclaving didecylmethylamine with gluconic acid and ethylene oxide in isopropanol to yield $[(C_{10}H_{21})_2CH_3N((CH_2)_2O)_nH]^+$ gluconate in Chem. Abstracts No. 109:124403x, while disinfectant solutions were prepared by exchanging a benzylammonium chloride with a chlorhexidine gluconate in Chem Abstracts No. 103:109954f.

Microbiocidal compositions which include quaternary ammonium compounds of the formula $R^1N^+R^2R^3R^4\ X^-$, wherein at least one of $R^1$, $R^2$, or $R^3$ is a $C_8$-$C_{30}$ alkyl or alkenyl group and the remainder of $R^1$, $R^2$ or $R^3$ is methyl, ethyl, $CH_2Ph$ or 4-pyridylmethyl; $R^4$ is methyl or ethyl; and X is an anion of an acid having a $C_7$ or greater hydrophobic group, were disclosed in Chem Abstracts Nos. 113:154360f and 113:153776j. Chem Abstracts No. 112:79768u discloses compounds of the formula $R^1R^2R^3R^4N^+X^-$, wherein $R^1$, $R^2$, and $R^3$ are methyl, ethyl, benzyl, 4-pyridinomethyl and at least one is $C_8$-$C_{30}$ alkyl or alkenyl; $R^4$ is methyl or ethyl; and X is a counter anion of acids having $C_7$ or greater hydrophobic groups. Dimethyldidecylammonium dodecylbenzenesulfonate was demonstrated to impart long term rot resistance to wood without causing rust, while the chloride salts of similar compounds were demonstrated to cause rust.

Patton et al., U.S. Patent No. 5,004,760, disclose polymeric foams incorporating various dialkyldimethylammonium carboxylates such as didecyldimethylammonium poly(ethylene/acetate) and the like.

Quaternary ammonium compounds (quats) are typically prepared by the reaction:

$$R^1R^2R^3N + R^4X \rightarrow R^1R^2R^3R^4NX \qquad (II)$$

wherein X is a halogen, a sulfate, a sulfo compound, or the like. When at least one of $R^1$, $R^2$, $R^3$, or $R^4$ is $C_{12}$ or longer, the product is an inert soap. Many of the inert soaps have biocidal activity against bacteria, fungi, algae, and related organisms.

Reaction (II) above is limited by the reactant $R^4X$ because $R^4$ must react with tertiary amines. For example, methyl chloride ($R^4X = CH_3Cl$) will react with a tertiary amine at less than 100°C to yield a quaternary compound $R_3N^+CH_3\ Cl^-$, while methanol or methyl acetate ($R^4X$=$CH_3OH$ or $CH_3COOCH_3$) will not, under similar reaction conditions.

General quaternary ammonium compounds with a sulfonate group are easily prepared either by the reaction of a sulfonate compound with a tertiary amine (III) or by a double exchange (IV).

$$R_3N + RSO_3CH_3 \rightarrow R_3NCH_3^+RSO_3^- \qquad (III)$$

$$R_3N^+CH_3\ Cl^- + RSO_3^-\ Na^+ \rightarrow R_3NCH_3^+\ RSO_3^- + NaCl \qquad (IV)$$

If trimethylamine is heated with carbon dioxide and methanol above 200°C and at 85 to 95 atmospheres, the carbonate quat, bis-tetramethylamanonium carbonate, is prepared. Industrial Organic Nitrogen Compounds, Astle Ed. p 66, Reinhold Inc, 1961. However, this reaction is limited to the methyl compound because higher homologs decompose to olefins by the Hofman elimination reaction. See, Organic Reactions, 11, Chptr. 5, 377, Krieger Publishing Co., 1975.

Chem Abstracts 110:212114 (1989) suggests that dimethyl carbonate will react with triethylamine in methanol in twelve hours at 115°C and under pressure to yield a methyl carbonate ester quat.

Chem Abstracts 114:24824 (1991) discloses that 6-hydroxyhexyldimethylamine reacts with dimethyl carbonate to yield a carbonate ester quat.

Quaternary ammonium hydroxides (hydroxy quats), an intermediate in the reaction scheme of the present invention, are currently prepared by the reaction of quaternary ammonium iodide with silver hydroxide (V).

$$RN^+(CH_3)_3\ I^- + AgOH \rightarrow RN^+(CH_3)_3OH^- + AgI \qquad (V)$$

However, this reaction is costly, and it is difficult to recover the silver reagent. See, Organic Reactions, 11:Chptr 5, pp. 376-377, Krieger Publishing Co., 1975.

In an olefin synthesis, it has been suggested to treat a quaternary salt with aqueous sodium or potassium hydroxide followed by pyrolysis in order to form the hydroxy quat and then to decompose the hydroxy quat directly. However, in this method the hydroxy quat is not isolated and the conditions for its preparation are undesirable. See, Organic Reactions, 11:Chptr 5, pp. 376-377, Krieger Publishing Co., 1975.

Talmon et al., Science, 221, 1047 (1983), have used an ion exchange resin to convert didecyldimethylammonium bromide to didecyldimethylammonium hydroxide (VI).

$$(C_{12}H_{25})_2(CH_3)_2N^+\ Br^- + \text{Ion Exchange Resin} \rightarrow$$

$$(C_{12}H_{25})_2(CH_3)_2N^+OH^-\ (VI) \qquad (VI)$$

However, 50 ml of ion exchange resin and two treatment steps were required to convert 3 grams of quaternary ammonium chloride to the corresponding hydroxide. Talmon et al. state that the hydroxy quat can be reacted with acids to make quats with different anions, and they have prepared didodecyldimethylammonium (DDDA) acetate, DDDA-formate, DDDA-propionate, DDDA-butyrate, DDDA-oxalate, DDDA-acrylate, DDDA-tartrate, DDDA-benzoate, and DDDA-octanoate. See also, Organic Synthesis, Collective Volume VI, 552, John Wiley Inc., 1988; Brady et al., J. Am.

Chem. Soc., 106:4280-4282, 1984; Brady et al., J. Phys. Chem., 90:9, 1853-1859, 1986; Miller et al., J. Phys. Chem, 91:1, 323-325, 1989; Radlinske et al., Colloids and Surfaces, 46:213-230, 1990.

Distearyldimethylammonium gluconate was prepared via ion exchange and subsequent reaction with an organic acid in Chem Abstracts No. 75:119170U. Miller et al, Langmuir, 4:1363 (1988) prepared ditetradecyldimethylammonium acetate by ion exchange from a bromide.

Alternatively, quaternary ammonium hydroxide compositions have been prepared by treating a haloquat in an electrochemical cell with special cation exchange diaphragms between the cells. The hydroxy quat collects at one electrode, and the halide collects at the other. Hydroxy quats, $R^1R^2R^3R^4N^+OH^-$, wherein the R groups were $C_1$-$C_4$, were treated with carboxylic acids to make asymmetric quats that were used as capacitor driving electrolytes. See, Japanese Patent Publication No. 02-106,915 and Awata et al., Chemistry, Letters, 371 (1985). Awata et al. placed carboxylic acids in the cathode cell to react with tetraethylammonium hydroxide as it was formed.

Japanese Patent Publication No. 01-172,363 discloses the preparation of relatively low yields of tetraethylammonium hydroxide by reacting triethylamine with diethyl sulfate, heating the resultant quat with sulfuric acid to yield the sulfate quat, and reacting the sulfate quat with barium hydroxide to yield the short chain quat, tetraethylammonium hydroxide, and barium sulfate.

Di $C_8$-$C_{12}$ alkyl quaternary ammonium hydroxides prepared by ion exchange were used as strong bases to digest animal tissue by Bush et al., French Patent Publication No. 1,518,427.

Akzo discloses that the addition of a metallic hydroxide to a quaternary ammonium chloride such as didecyldimethylammonium chloride, in an aqueous medium, results in an equilibrium mixture of quaternary ammonium chloride and quaternary ammonium hydroxide (VII). This reaction can be driven to the right by the use of isopropanol as a solvent.

$$(R_4N)Cl + KOH \rightleftarrows (R_4N)OH + KCl \qquad (VII)$$

Akzo further discloses that the addition of a soap to a quaternary ammonium chloride yields a quaternary ammonium carboxylate (VIII).

$$(R_4N)Cl + R^1COONa \rightarrow (R_4N)\,(OOCR^1) + NaCl \qquad (VIII)$$

Jordan et al., U.S. Patent No. 3,281,458, disclose the preparation of dioctadecyldimethylammonium humate, ditallowdimethylammonium humate, dipentadecyldimethylammonium humate, and didodecyldimethylammonium humate by reacting humic acid, lignite, aqueous sodium hydroxide and a chloride quat.

Finally, Nakama et al., J.A.C.O.S., 67:717 (1990) report the interaction between anionic and cationic surfactant and particularly sodium laurate and stearyltrimethylammonium chloride, while Linderborg, U.S. Patent No. 4,585,795, disclose the use of synergistic mixtures of the alkali metal salt of certain biocidal organic acids, quaternary ammonium chlorides, and alkyl-pyridinium chlorides as control agents for short-term protection of timber against sapstain fungi and mildew.

Consequently, efforts have been directed to develop a safe, efficient and expedient method to prepare quaternary ammonium compounds that do not require potentially hazardous metal additives to treat wooden substrates effectively.

It has been discovered that di $C_8$-$C_{12}$ alkyl quaternary ammonium carbonate quats are useful in wood preservative systems as they have improved leaching resistance, particularly without the use of the commonly used metal stabilizers or couplers, arsenic, chromium, copper, and zinc or combinations thereof.

Additionally, di $C_8$-$C_{12}$ alkyl quaternary ammonium carboxylates can be incorporated into metal-free wood preservative systems.

Di $C_8$-$C_{12}$ alkyl carboxylate quats above, can be prepared by various methods from $C_8$-$C_{20}$ alkyl quaternary ammonium chloride (chloride quat(s)) starting materials, including by indirect synthesis through $C_8$-$C_{20}$ alkyl quaternary ammonium hydroxide and $C_8$-$C_{20}$ alkyl quaternary ammonium carbonate intermediates or by direct synthesis.

The di $C_8$-$C_{12}$ alkyl carbonate including those prepared by the methods above, are useful as wood preservatives, as they have improved leaching resistance, particularly without the use of the commonly used metal stabilizers or couplers, arsenic, chromium, copper, and zinc or combinations thereof.

Typically, quaternary ammonium compounds migrate or leach from wood under wet conditions, however. Common waterproofing compositions have not proven compatible with the quaternary ammonium compounds typically used in the industry, and therefore, they are not commonly used to hinder the leaching of these quats.

Typical waterproofers are waxes, lower molecular weight polyolefins, or dispersions or solutions thereof in

hydrocarbon solvents. However, quaternary compositions, including those useful in the present invention, typically are water soluble. Generally, they are not soluble in these typical waterproofer solvent systems and are not compatible with emulsified or dispersed waterproofers.

It has now been disccvered that di $C_8$-$C_{12}$ alkyl, quaternary ammonium hydroxides, carbonates, carboxylates, and borates including those prepared by the methods described herein, are compatible with newly discovered polyhydroxyl or polyetherhydroxyl esters of fatty acids or polyether hydroxide waterproofers. Waterproofing and wood preservative systems prepared from the waterproofers or waterproofers and quats described herein exhibit enhanced resistance to leaching and meet waterproofing standards for heavy duty, ground, or millwork applications.

## BRIEF DESCRIPTION OF THE DRAWINGS

Figure 1A is a graphic comparison of leaching of a wood preservative system according to the present invention and a wood preservative system of the prior art.
Figure 1B is an enlarged segment of the graph of Figure 1A.
Figure 2A is a graphic comparison of leaching of a wood preservative system according to the present invention and a wood preservative system of the prior art.
Figure 2B is an enlarged segment of the graph of Figure 2A.
Figure 3A is a graphic comparison of leaching of preservative systems according to the present invention and wood preservative systems of the prior art.
Figure 3B is an enlarged segment of the graph of Figure 3A.
Figure 3C is a graphic comparison of leaching of preservative systems according to the present invention and alternative wood preservative systems.
Figure 4A is a graphic comparison of leaching of waterproofer containing wood preservative systems according to the present invention and wood preservative systems without waterproofer.
Figure 4B is an enlarged section of Figure 4A.

## SUMMARY OF THE INVENTION

According to the first aspect of the invention there is provided a wood preservative system comprising (a) a biocidal effective amount of at least one di $C_8$-$C_{12}$ alkyl quaternary ammonium hydroxide in the wood substrate and (b) a solvent, wherein said wood preservative system is metal-free.

According to a second aspect of the present invention there is provided a wood preservative composition comprising

(a) at least one di-($C_8$-$C_{12}$ alkyl) quaternary ammonium carbonate having the formula

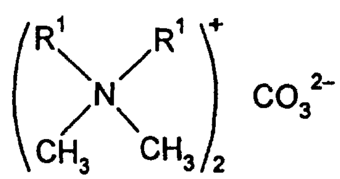

wherein $R^1$ is a $C_8$-$C_{12}$ alkyl group; and
(b) (1) at least one di-($C_8$-$C_{12}$ alkyl) quaternary ammonium bicarbonate having the formula

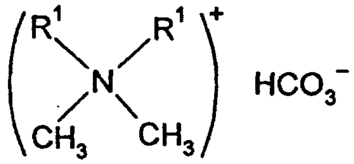

wherein $R^1$ is the same or different $C_8$-$C_{12}$ alkyl group as in (a); or

(2) at least one di-($C_8$-$C_{12}$ alkyl) quaternary ammonium metal carbonate having the formula

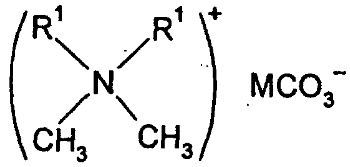

wherein $R^1$ is the same or different $C_8$-$C_{12}$ alkyl group as in (a) or (1) and M is a non-coupler metal, or
(3) a combination of (1) and (2);
said composition being metal coupler-free.

According to a further aspect of the present invention there is provided a wood preservative system comprising (A) a biocidal effective amount of at least one di $C_8$-$C_{12}$ alkyl quaternary ammonium carbonate having the formula

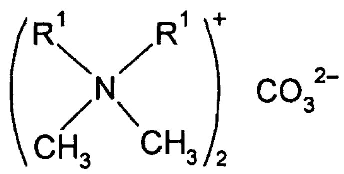

wherein $R^1$ is a $C_8$-$C_{12}$ alkyl group and (B) a solvent, wherein said system is metal coupler free.

Quaternary Ammonium Hydroxide

Quaternary ammonium hydroxides suitable for use in the present invention have the formula

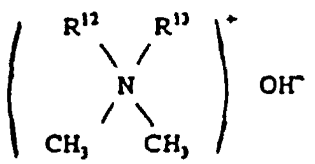

(XVI)

wherein $R^{13}$ and $R^{12}$ is a $C_8$-$C_{12}$ alkyl group.

Special mention is made of hydroxy quats wherein $R^{12}$ is a $C_8$ alkyl, $C_9$ isoalkyl, $C_{10}$ alkyl, $C_{12}$ alkyl, and $R^{13}$ is a $C_{10}$ alkyl group. Most preferred hydroxy quats are didecyldimethylammonium hydroxide wherein $R^{12}$ and $R^{13}$ are a $C_{10}$ alkyl group and most preferably an n-$C_{10}$ alkyl group.

Didecyldimethylammonium hydroxide, when observed in a 70 to 80 percent by weight solution in a 50 percent by weight alcohol/50 percent by weight water solvent, is a yellow/orange liquid. This formulation has a flash point of about 56.7 °C (134 °F), and it is a highly alkaline material that reacts with the phenolic OH of lignin.

Quaternary ammonium hydroxides useful in the present invention are preferably prepared according to the reaction illustrated below.

The method of the present invention provides increased yields of di $C_8$-$C_{12}$ alkyl quaternary ammonium hydroxide, when compared with conventional production methods. Although it was previously believed that the reaction of the chloride quat salt with a metal hydroxide to yield quaternary ammonium hydroxide and metal chloride was an equilibrium reaction (VII) or could be driven to the right by the use of branched solvents, it has now been discovered that by selection of the proper reactants, reaction medium, and/or reaction conditions (including reactant amounts), the reaction can be driven well past equilibrium to yield unprecedented greater amounts of $C_8$-$C_{12}$ alkyl quaternary ammonium hydroxide.

Most preferred hydroxy quats are di n-$C_8$-$C_{12}$ alkyl quaternary ammonium hydroxide, didecyldimethylammonium hydroxide, and di-n-decyldimethylammonium hydroxide.

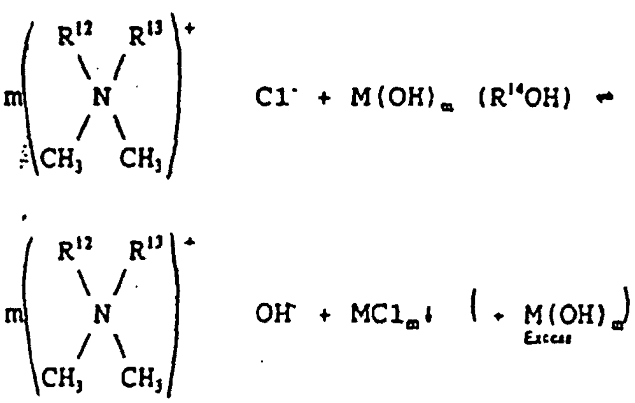

(XXV)

wherein $R^{14}$ is a straight chain $C_1$-$C_4$ alkyl group,

M is a mono-, di-, or trivalent metal; and m is one if is monovalent, two if M is divalent, and three if M is trivalent, and $R^{12}$ is the same as $R^{13}$, i.e. a $C_8$-$C_{12}$ alkyl group.

Many di $C_8$-$C_{12}$ alkyl quatemary ammonium chlorides are suitable reactants and didecyldimethylammonium chloride, and particularly, di-n-decyldimechylammonium chloride is most preferred. The selections of the $R^{12}$ and $R^{13}$ substituents of the chloride quat reactant are determinative of the hydroxy quat product.

Special mention is also made of processes wherein R" is a $C_8$ alkyl, $C_9$ isoalkyl, $C_{10}$ alkyl, $C_{12}$ alkyl, and $R^{13}$ is a $C_{10}$ alkyl or$C_{12}$ alkyl, group.

The metal hydroxide reactant is a mono-, bi-, or trivalent metal hydroxide, preferably a monovalent metal hydroxide, and most preferably an alkali metal hydroxide such as sodium hydroxide or potassium hydroxide. Special mention is made of potassium hydroxide. The metal chloride reaction product will precipitate and is easily removed, i. e. by filtration or the like, yielding a hydroxy quat/solvent reaction product. The hydroxy quat can be separated therefrom by drying or the like.

The reaction is conducted in a solvent which comprises a $C_1$-$C_4$ normal alcohol. Preferably, the solvent is ethanol, and most preferably, anhydrous ethanol.

The amount of metal hydroxide reactant typically is a stoichiometric amount with respect to the quaternary ammonium chloride reactant. Therefore, on a theoretical basis and if the reaction were complete and unequilibrated, there would be no excess of metal hydroxide reactant upon completion of the reaction. In practice, yield when using a stoichiometric amount of metal hydroxide reactant will range from about 65% to about 95%, but will vary, dependent in part upon the particular metal hydroxide reactant.

Yield can be further improved over conventional methods by utilization of a stoichiometric excess of metal hydroxide ranging from about 2% to about 20% excess. If an excess of metal hydroxide is used, yield will be increased to from about 95% to about 99%, again varying as above.

The unreacted metal hydroxide is soluble in the hydroxy quat/solvent mixture. Any excess or unreacted metal hydroxide should be removed after the reaction is completed, and is preferably precipitated by subsequent reaction with carbon dioxide to yield the corresponding metal carbonate. The carbonate is insoluble in the hydroxy quat/solvent mixture and is easily removed, i.e. by filtration or the like. Alternatively, a solid metal bicarbonate, in which the metal corresponds to the metal of the metal hydroxide, can be added and slurried with the hydroxy quat/solvent mixture. The soluble metal hydroxide reacts with solid bicarbonate to yield the insoluble metal carbonate. The metal carbonate does not react further with the hydroxy quat.

Mixing, adding, and reacting of the components in the preparation of these hydroxy quats can be accomplished by conventional means known to those of ordinary skill in the art. The order of addition of reactants or solvent does not affect the process. Reactants and/or solvent can be added sequentially or simultaneously in any suitable reaction vessel.

Typically, the reactants and solvent will be stirred and heated to from about 20°C to about 70°C and held at that temperature for a period of from about 1 hour to about 5 hours. The reaction mixture is then cooled, first to room temperature and then to about 0°C where it is held for about 1 hour to about 2 hours. Any precipitated metal chloride is collected as is known in the art, i.e. such as by filtration.

Alternatively, the reactants and solvent can be stirred at a slightly elevated temperature, i.e. from about 20°C to about 40°C, to yield the hydroxy quat/solvent mixture. Hydroxy quat can be separated as above.

Di $C_8$-$C_{12}$ alkyl quaternary ammonium hydroxides, and particularly those prepared by the method of the present invention, can be formulated as metal-free wood preservative systems. These systems include biocidal effective amounts of at least one hydroxy quat and a suitable solvent, including aqueous and non-aqueous solvents. Preferably, the solvent is an aqueous solvent including, but not limited to, water, aqueous alcohol such as ethanol, ammonia water, and the like, or a combination of any of the foregoing.

Although other conventional additives may be added as required for application to different substrates and for different uses as known to those of ordinary skill in the art, metal stabilizers are not required and, in fact, are not recommended to inhibit leaching of the quat from the substrate. Accordingly, wood substrates, such as lumber, timber, or the like, can be treated with preservative systems which comprise the above hydroxy quat(s) diluted in a suitable solvent as above.

The amount of di $C_8$-$C_{12}$ alkyl quaternary ammonium hydroxide used to treat the substrate is a biocidal effective amount, i.e. that amount effective to inhibit the growth of or to kill one or more organism that causes wood rot, to inhibit sap staining, or any combination thereof. Such organisms include, but are not limited to, Trametes viride or Trametes versicolor, which cause a white rot; Goeophyllium trabeum, which causes a brown rot; and Aspergillus niger, which causes sap stain/mold.

Typically, a wood preservative system will comprise from 0.1 to 5 parts by weight of the hydroxy quat and from 95 to 99.9 parts by weight of solvent based upon 100 parts by weight of quat and solvent combined. Most'preferably, the wood preservative system of the present invention will comprise from about 1 to about 2 parts by weight of hydroxy quat and from about 98 to about 99 parts by weight of solvent on the same basis.

Treatment of the substrate is accomplished by any means known to those of ordinary skill in the art including, but not limited to dipping, soaking, brushing, pressure treating or the like. The length of treatment required will vary according to treatment conditions, the selection of which are known to those skilled in the art.

The wood preservative systems of the present invention display greater resistance to leaching than wood preservatives currently used in the industry. Resistance to leaching is defined as retention of,a biocidal effective amount, and preferably at least about 2% by weight, of hydroxy quat in the substrate over a prolonged period of at least about 100 hours and preferably about 350 hours. Applicants hypothesize, without being bound by any theory, that the hydroxide quat reacts or complexes with the woody matrix of the substrate, thereby "fixing" it in the substrate. It is also believed that the long chain hydroxy quats and the wood preservative systems that include such quats enhance waterproofing properties of the treated substrates.

Quaternary Ammonium Carbonate

Ammonium carbonates suitable for use in the present invention have the formula

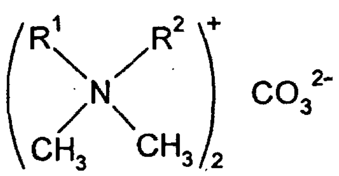

(XI)

wherein. $R^1$ and $R^2$ are the same $C_8$-$C_{12}$ alkyl group.

Most preferred carbonate quats are didecyldimethylammonium carbonate wherein $R^1$ and $R^2$ are a $C_{10}$ alkyl group and preferably an n-$C_{10}$ alkyl group. Didecyldimethylammonium carbonate, when observed as a 70-80 percent by weight solution is a yellow/orange liquid that has a slightly fruity odor. This formulation has a flash point of about 71 °C (160 °F), and it reacts with carboxyl containing compounds.

One or more of these carbonate quats alone or in combination with the corresponding bicarbonate quat(s) and/or metal carbonate salt(s), preferably potassium carbonate salt, can be formulated in the present wood preservative systems.

The stability, and particularly the thermal stability, of carbonate quats is superior to that of hydroxy quats, making these carbonate quats suitable for concentrating and as stock intermediates for further processing.

One or more of these carbonate quats alone or in combination with the corresponding bicarbonate quat(s) and/or metal carbonate salt(s), preferably potassium carbonate salt, can be formulated as metal coupler-free wood preservative systems. These systems include biocidal effective amounts of at least one carbonate quat and a suitable solvent, including aqueous and non-aqueous solvents. Preferably, the solvent is an aqueous solvent including, but not limited to, water, aqueous alcohol such as aqueous ethanol, ammonia water, and the like, or a combination of any of the foregoing.

Although other conventional additives may be added as required for application to different substrates and for different uses as known to those of ordinary skill in the art, metal stabilizers are not required and, in fact, are not

recommended to inhibit leaching of the quat from the substrate. Accordingly, wood substrates, such as lumber, timber, and the like, can be treated with metal coupler-free preservative systems which comprise the above carbonate quat (s) diluted in a suitable solvent as above.

The amount of di $C_8$-$C_{12}$ alkyl quaternary ammonium carbonate(s) used to treat the substrate is a biocidal effective amount, i.e. that amount effective to inhibit the growth of or to kill one or more organism that causes wood rot, to inhibit sap stain, or a combination thereof. Such organisms include, but are not limited to, Trametes viride or Trametes versicolor, which cause a white rot; Goeophyllium trabeum, which causes a brown rot; and Aspergillus niger, which causes sap stain/mold.

Typically, a wood preservative system will comprise from 0.1 to 5 parts by weight of the carbonate quat(s) and from 95 to 99.9 parts by weight of solvent based upon 100 parts by weight of quat and solvent combined. Most preferably, the wood preservative system of the present invention will comprise from about 1 to about 2 parts by weight of carbonate quat(s) and from about 98 to about 99 parts by weight of solvent on the same basis.

Treatment of the substrate is accomplished by any means known to those of ordinary skill in the art including, but not limited to, dipping, soaking, brushing, pressure treating, or the like. The length of treatment required will vary according to treatment conditions, the selection of which are known to those skilled in the art.

These metal coupler-free preservative systems display greater resistance to leaching than wood preservatives currently used in the industry. Resistance to leaching is defined as retention of a biocidal effective amount, and preferably at least about 2% by weight, of carbonate quat(s) in the substrate over a prolonged period of at least about 100 hours and preferably about 350 hours. Applicants hypothesize, without being bound by any theory, that the carbonate quat reacts or complexes with the woody matrix of the substrate, thereby "fixing" it in the substrate. It is also believed that the long chain carbonate quat(s) and the wood preservative systems that include such quats enhance waterproofing properties of treated substrates.

Although certain carbonate quats can be prepared by a variety of methods, applicants have discovered an indirect synthesis method that can be used to prepare a variety of di $C_8$-$C_{12}$ alkyl quaternary ammonium carbonate compounds, preferably didecyldimethylammonium carbonate.

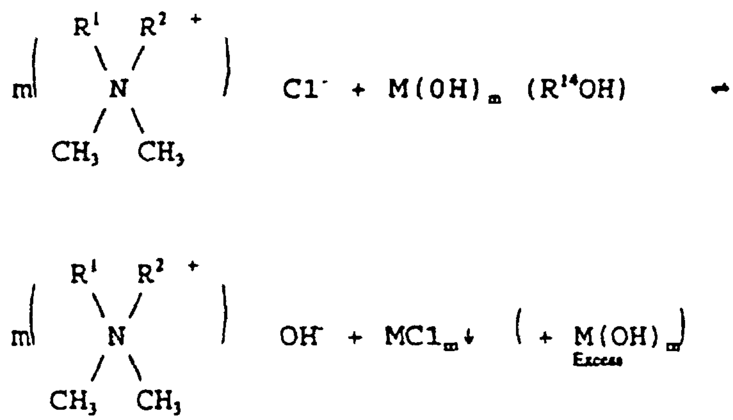

(XXVI)

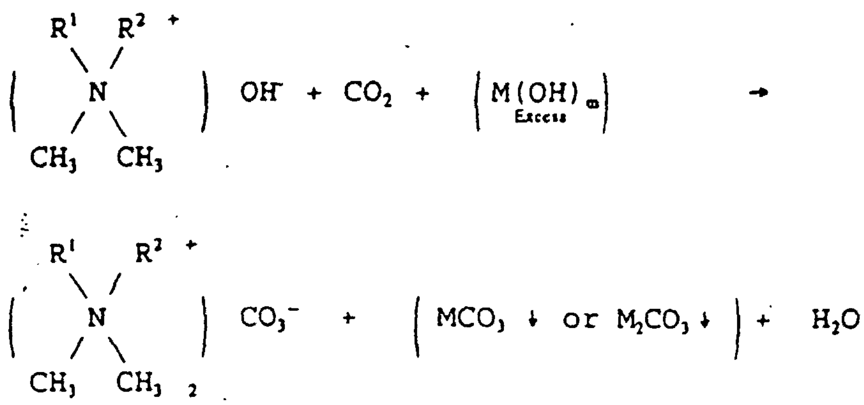

(XXVII)

wherein
$R^1$ is the same as $R^2$ and $R^1$ is a $C_8$-$C_{12}$ alkyl group; $R^{14}$ is a straight chain $C_1$-$C_4$ alkyl group; M is a mono-, bi-, tri-valent metal, preferably a mono-valent metal, and most preferably an alkali metal; and m is 1 if M is mono-valent, 2 if M is di-valent, and 3 if M is tri-valent.

A di $C_8$-$C_{12}$ alkyl, quaternary ammonium chloride is used as a starting material and is reacted with a metal hydroxide to yield a di $C_8$-$C_{12}$ alkyl, quaternary ammonium hydroxide intermediate. The hydroxy quat intermediate(s) and any excess metal hydroxide are then reacted with carbon dioxide to yield the carbonate quat(s) and the metal carbonate.

Many di $C_8$-$C_{12}$ alkyl quaternary ammonium chlorides are suitable reactants to prepare the intermediate hydroxy quat and are described above. The selections of the $R^1$ and $R^2$ substituents of the chloride quat reactant are determinative of the hydroxy quat intermediate, and therefore, of the carbonate quat product.

The metal hydroxide reactant is also as described above.

The metal chloride first step reaction product will precipitate and is easily removed, i.e. by filtration or the like, yielding a hydroxy quat/solvent reaction product. The hydroxy quat can be separated therefrom by drying or the like, if desired.

The first reaction (XXVI) is conducted in a solvent as described above, and the amount of metal hydroxide reactant is as described above.

Hydroxy quat and any unreacted metal hydroxide are then reacted with at least a stoichiometric equivalent of carbon dioxide to yield the quaternary ammonium carbonate(s), and if any unreacted metal hydroxide is present, the metal carbonate(s). The conversion of the metal hydroxide to the metal carbonate is the preferred reaction of the two carbonations and will proceed more rapidly. The metal carbonate will precipitate and.can be separated easily, i.e. by filtration or the like, leaving the stable carbonate quat(s) or carbonate quat(s)/solvent reaction product.

The carbonation step can also produce the bicarbonate quat or the metal carbonate quat as byproducts. The carbonate quat alone or in combination with the bicarbonate quat and/or the metal carbonate quat are suitable for use in the metal coupler-free wood preservative systems of the present invention. These carbonate quats or carbonate/bicarbonate/metal carbonate compositions, do not require a metal coupler for stabilization in a wood substrate. Completely metal-free wood preservative systems are preferred. However, if a metal carbonate quat is included in the system, preferably the metal is not a metal currently used as a coupler, and most preferably, it is an alkali metal and does not pose environmental or corrosion hazards or concerns.

Mixing, adding, and reacting of the components in the preparation of these carbonate quats can be accomplished by conventional means known to those of ordinary skill in the art. The order of addition of reactants or solvent in any individual step does not affect the process. Reactants and/or solvent can be added sequentially or simultaneously in any suitable reaction vessel. For example, the metal hydroxide may be dissolved in alcohol and the resultant mixture added to the chloride quat or the chloride quat may be dissolved in alcohol and the metal hydroxide added to the resultant mixture.

The carbon dioxide is generally bubbled for a suitable period known to those of ordinary skill in the art through the hydroxy quat/solvent supernatant after the metal chloride precipitate has been separated. Alternatively, the carbon dioxide can be added as solid dry ice directly to the hydroxy quat. Typically, this time varies from about 0.5 hour to about 1 hour at ambient temperature. Any precipitated metal carbonate is collected as is known in the art, i.e., such as by filtration.

Solvents

The preservative systems of the present invention include a suitable solvent including aqueous and non-aqueous solvents. Preferably, the solvent is an aqueous solvent including, but not limited to, water, aqueous alcohol, ammonia water, aqueous acetic acid, and the like, or a combination of any of the foregoing. Organic solvents may also be used. These include, but are not limited to, mineral spirits-based solvents and the like.

## DESCRIPTION OF THE PREFERRED EMBODIMENTS

The following examples illustrate the invention without limitation. All parts and percentages are given by weight unless otherwise indicated.

Quaternary compounds are quantified by two phase titration with sodium laurylsulfate and an indicator. The mixture is buffered to a pH of 10.

Swell index is calculated as

$$\left( \frac{\text{(Swell of Control - Swell of Sample)}}{\text{Swell of Control}} \right) \times 100$$

## PREPARATION OF HYDROXY QUATS

Example 1 - Stoichiometric Amount of Metal Hydroxide

180 grams (0.4 moles) of 80% didecyldimethylammonium chloride in 20% ethanol water (144 grams of DDAC), 180 ml of absolute denatured ethanol (denatured with methanol/isopropanol), and 26 grams (0.4 mole) of 85% potassium hydroxide pellets (22.1 grams of KOH) were mixed in a flask that was purged with nitrogen and equipped with a heating mantle and a magnetic stirrer. The mixture was stirred and heated at 60-70°C for three hours. The mixture was then allowed to cool to room temperature and finally cooled to 0°C for at least one hour.

Potassium chloride precipitated, and the precipitate was collected on a vacuum filter. The solid was washed with cold ethanol and subsequently was dried, yielding 30 grams of dry potassium chloride. The quat solution was concentrated in a vacuum to about 75% active bases.

Yield was 180 grams of product containing 138 grams of didecyldimethylammonium hydroxide.

Example 2

The procedure of Example 1 was followed, but the mixture was stirred mechanically at 50°C for one hour. Potassium chloride precipitated, and the precipitate was collected on a vacuum filter. The solid was washed with cold ethanol and subsequently was dried, yielding 30 grams of dry potassium chloride.

Yield was 180 grams of product containing 138 grams of didecyldimethylammonium hydroxide.

Example 3

0.022 mole of 85% potassium hydroxide pellets (1.23 grams of KOH) was added to 0.022 mole of 80% didecyldimethylammonium chloride in 20% ethanol/water ( 8 grams of DDAC) dissolved in 10 ml of ethanol. The resultant mixture was stirred and heated to 70°C and held at this temperature for one-half hour. The pellets dissolved, and a fine precipitate formed. The mixture was then cooled and chilled to 0°C. The precipitated solid was collected on a filter and washed with cold ethanol. The filtrate was concentrated to yield a yellow/orange oil with a slight amine odor.

Results are summarized in Table 1.

Comparative Example 3A

The procedure of Example 3 was followed substituting isopropanol for the ethanol.

Results are illustrated in Table 1.

Example 4

0.022 mole of 85% potassium hydroxide pellets ( 1.23 grams of KOH) was added to 0.022 mole of 80% didecyldimethylammonium chloride in 20% ethanol/water (8 grams of DDAC) dissolved in 10 ml of propanol. The

resultant mixture was stirred and heated to 80°C and held at this temperature for one hour. The pellets dissolved, and a fine precipitate formed. The mixture was then cooled and chilled to 0°C. The precipitated solid was collected on a filter and washed with cold ethanol. The filtrate was concentrated to yield a yellow/orange oil with a slight amine odor.

Results are illustrated in Table 1.

Example 5

The procedure of Example 3 was followed substituting sodium hydroxide for the potassium hydroxide.

Results are illustrated in Table 1.

Comparative Example 5A

The procedure of Comparative Example 3A was followed substituting sodium hydroxide for the potassium hydroxide.

Results are illustrated in Table 1.

Example 6

The procedure of Example 4 was followed substituting sodium hydroxide for the potassium hydroxide.

Results are illustrated in Table 1.

TABLE 1

| Preparation of Didecyldimethylammonium Hydroxide from Stoichiometric Amounts of Reactants | | | | | | |
|---|---|---|---|---|---|---|
| Example | 3 | 3A | 4 | 5 | 5A | 6 |
| Hydroxide | KOH | KOH | KOH | NaOH | NaOH | NaOH |
| Solvent | Ethanol | Isopropanol | n-propanol | Ethanol | Isopropanol | n-propanol |
| Conversion % | 96 | 86 | 95 | 81 | 66 | 83 |

Examples 3-6 when compared with Comparative Examples 3A and 5A demonstrate that the use of a normal $C_1$-$C_4$ alcohol as a reaction medium enhances conversion of the chloride quat to the hydroxy quat. Furthermore, a comparison of examples 3 and 4 with Examples 5 and 6 illustrates the increase in conversion by the use of the preferred metal hydroxide, potassium hydroxide.

Stoichiometric Excess of Metal Hydroxide

Example 7

A nitrogen purged reactor equipped with a heating mantle and a magnetic stir bar was charged with 0.4 mole of 80% didecyldimethylammonium chloride (144 grams of DDAC) in 20% ethanol/water, 180 ml of ethanol, and 0.49 mole of 85% potassium hydroxide ( 27.5 grams of KOH) pellets. The mixture was heated at 60-70°C for 3 hours, allowed to cool to room temperature, and then cooled to 0°C for about one hour to precipitate potassium chloride. The precipitate was collected on a vacuum filter, and the solid was washed with cold ethanol. Potassium chloride yield was 30.8 grams.

The supernatant solution, which contained the hydroxy quat and 0.09 moles of excess potassium hydroxide, was stirred with 2 grams (0.045 moles) of carbon dioxide gas (from dry ice). The mixture was kept cold for an hour and then was vacuum filtered to remove 7.2 grams (theoretical 6.2 grams) of potassium carbonate.

Conversion percentage to the hydroxy quat was determined to be 99%.

Treatment of Wood Substrates

Example 8

End grain pine wafers were weighed and then soaked with didecyldimethylammonium hydroxide until a weight gain of 30% was observed.

The treated wafers were then placed in water and weighed periodically to determine resistance to leaching.

Results are illustrated in Figures 1A and 1B.

Comparative Example 8A

The procedure of Example 8 was followed substituting didecyldimethylammonium chloride for the didecyldimethylammonium hydroxide.
Results are illustrated in Figures 1A and 1B.
Figures 1A and 1B illustrate that the hydroxy quat resists leaching for extended periods while the chloride quat leaches to levels of 1% or less in a relatively short period.

Example 9

A 25.4×1.27×1.91 $cm^3$ (10"×0.5"×0.75") piece of ponderosa pine was equilibrated, weighed, and heated for two hours at 60°C. The wood was treated with a treating solution of 2% didecyldimethylammonium hydroxide in water by heating in the solution at 60°C to 80°C for one hour, cooling and standing overnight, and then being subjected to a second warm to cool cycle. The samples were allowed to dry to constant weight, and the uptake was determined by comparing starting and finishing weights.
The samples were then heated for two hours at 60°C, and the weight of the warm treated samples was compared to the over dried sticks before treatment.
Results are illustrated in Table 2.

Comparative Example 9A

The procedure of Example 9 was followed, omitting the didecyldimethylammonium hydroxide from the treating solution.
Results are illustrated in Table 3.

Comparative Example 9B

The procedure of Example 9 was followed, substituting didecyldimethylammonium chloride for the didecyldimethylammonium hydroxide.
Results are illustrated in Table 2

TABLE 2

| Weight Uptake from Quat Solutions | | | |
|---|---|---|---|
| Example | 9 | 9A | 9B |
| Solvent | Water | Water | Water |
| Quat | Hydroxide | - | Chloride |
| Weight Uptake (%) | 2.5 | 0.4 | 0.6 |

Example 9 when compared with Comparative Examples 9A and 9B, respectively, illustrate the ability of the hydroxy quats prepared according to the present invention to be applied to wood substrates. The hydroxy quat is absorbed better than the chloride quat in water, and is absorbed similarly to the art accepted chloride quat in ammonia/water. However, the hydroxy quats can be used without metal coupling agents in treating wood substrates.

Example 10

A piece of wood was treated according to the procedure of Example 9. The piece of wood was then soaked in water at room temperature for 24 hours, dried to constant weight, and weighed to determine how much chemical remained. The piece of wood was soaked for 96 additional hours (120 hours total), dried to constant weight, and weighed to determine the leaching of quat from the treated wood. The water was changed several times during this period.
Results are illustrated in Table 3.

Comparative Example 10A

A piece of wood was treated according to the procedure of Comparative Example 9A. The piece of wood was then soaked according to the procedure of Example 10.
Results are illustrated in Table 3.

Comparative Example 10B

A piece of wood was treated according to the procedure of Comparative Example 9B. The piece of wood was then soaked according to the procedure of Example 10.
Results are illustrated in Table 3.

TABLE 3

| Leaching of Quat | | | |
|---|---|---|---|
| Example | 10 | 10A | 10B |
| Solvent | Water | Water | Water |
| Quat | Hydroxide | | Chloride |
| Weight Uptake (%) | 2.5 | 0.4 | 0.6 |
| Retained Quat at 24 Hours (Absolute %/Relative %) | 2.3/92 | -0.2/- | 0.5/83 |
| Retained Quat at 120 Hours (Absolute %/Relative %) | 1.8/72 | -0.2/- | 0.4/67 |

Example 10, when compared with Comparative Examples 10A and 10B, demonstrates the improved retention properties of hydroxy quats prepared according to the present invention over conventional chloride quats, particularly in the absence of metal stabilizers.

Preparation of Carbonate Quats

Example 11

180 grams (0.4 moles) of 80% didecyldimethylammonium chloride in 20% ethanol water (144 grams DDAC), 180 ml of absolute denatured ethanol (denatured with methanol/isopropanol), and 32 grams (0.49 mole) of 85% potassium hydroxide pellets (27 grams KOH) were mixed in a flask that was purged with nitrogen and equipped with a heating mantle and a magnetic stirrer. The mixture was stirred and heated at 60-70°C for three hours. The mixture was then allowed to cool to room temperature and finally cooled to 5°C.
Potassium chloride precipitated, and the precipitate was collected on a vacuum filter. The solid was washed with cold ethanol and subsequently was dried, yielding 31 grams (calculated yield 29.6 grams) of dry potassium chloride.
The ethanolic solution of the hydroxy quat containing about 0.09 mole of unreacted KOH, was stirred while 50 grams of carbon dioxide (from sublimed carbon dioxide) were bubbled over one half hour. The resultant mixture was then filtered to remove 7.2 grams of potassium carbonate (6.2 grams calculated), and the filtrate was concentrated to yield an orange/brown liquid with 80-85% carbonate quat in water/ethanol and less than 0.1% chloride quat having a product with 98 to 99% exchanged quat purity.

Example 12

180 grams (0.4 moles) of 80% didecyldimethylammonium chloride in 20% ethanol water (144 grams DDAC), 180 ml of absolute denatured ethanol (denatured with methanol/isopropanol), and 32 grams (0.49 mole) of 85% potassium hydroxide pellets (27 grams KOH) were mixed in a flask that was purged with nitrogen and equipped with a heating mantle and a magnetic stirrer. The mixture was heated to 50°C and stirred for one hour.
Potassium chloride precipitated, and the precipitate was collected on a vacuum filter. The solid was washed with cold ethanol and subsequently was dried, yielding 31 grams (calculated yield 29.6 grams) of dry potassium chloride.
The ethanolic solution of the hydroxy quat containing about 0.09 mole of unreacted KOH, was stirred while 50 grams of carbon dioxide (from sublimed carbon dioxide) were bubbled over one half hour. The resultant mixture was then filtered, and the filtrate was concentrated to yield an orange/brown liquid. Yield was similar to that of Example 11.

Treatment of Wood Substrates

Example 13

End grain pine wafers were weighed and then soaked with didecyldimethylammonium carbonate until a weight gain of 30% was observed.

The treated wafers were then placed in water and weighed periodically to determine resistance to leaching.

Results are illustrated in Figures 1A and 1B.

Comparative Example 13A

The procedure of Example 13 was followed substituting didecyldimethylammonium chloride for the didecyldimethylammonium carbonate.

Results are illustrated in Figures 2A and 2B.

Figures 2A and 2B illustrate that the carbonate quat resists leaching for extended periods while the chloride quat leaches to levels of 1% or less in a relatively short period.

Examples 14 and 15 and Comparative

Examples 14A, 14B, 15A and 15B

A 25.4×1.27×1.91 $cm^3$ (10"×0.5"×0.75") piece of ponderosa pine was equilibrated, weighed, and heated for two hours at 60°C. The wood was treated with a treating solution of 2% didecyldimethylammonium carbonate in water solvent by heating in the solution at 60°C to 80°C for one hour, cooling and standing overnight, and then being subjected to a second warm to cool cycle. The samples were allowed to dry to constant weight, and the uptake was determined by comparing starting and finishing weights.

The samples were then heated for two hours at 60°C, and the weight of the warm treated samples was compared to the oven dried sticks before treatment.

Additional examples were prepared either omitting the carbonate quat, substituting a chloride quat, or using 1% quat in a 3% aqueous ammonia solvent.

Formulations and results are illustrated in Table 4.

TABLE 4

| Weight Uptake from Quat Solutions | | | | | | |
|---|---|---|---|---|---|---|
| Example | 14 | 14A | 14B | 15 | 15A | 15B |
| Solvent | Water | Water | Water | 3% Ammonia | 3% Ammonia | 3% Ammonia |
| Quat | Carbonate | - | Chloride | Carbonate | - | Chloride |
| Weight Uptake (%) | 1.8 | -0.4 | 0.6 | 1.6 | -0.6 | 2.0 |

Examples 14 and 15, when compared with Comparative Examples 14A, 14B, 15A, and 15B respectively, illustrate the ability of the carbonate quats of the present invention to be applied to wood substrates. The carbonate quat is absorbed better than the chloride quat in water, and is absorbed similarly to the art accepted chloride quat in ammonia/water. However, the carbonate quats can be used without metal coupling agents in treating wood substrates.

Examples 16 - 19 and Comparative Examples 16A, 16B, 19A and 19B

A piece of wood was treated according to the procedure of Example 14. The piece of wood was then soaked in water at room temperature for 24 hours, dried to constant weight, and weighed to determine how much chemical remained. The piece of wood was soaked for 96 additional hours (120 hours total), dried to constant weight, and weighed to determine the leaching of quat from the treated wood. The water was changed several times during this period.

Additional examples were prepared with different quat concentrations, different anions, and different solvents.

Formulations and results are illustrated in Table 5.

It is pointed out that the embodiments of the following examples 20-26, 49-74, 79-91 and 96-98 are merely disclosed for illustrative purposes and are not part of the claimed subject-matter.

TABLE 5

Leaching of Quat

| Example | Solvent | Quat | Weight Uptake (%) | Retained Quat at 24 Hours (Absolute %/ Relative %) | Retained Quat at 120 Hours (Absolute %/ Relative %) |
|---|---|---|---|---|---|
| 16 | Water | 2% Carbonate | 1.8 | 2/110 | 1.6/80 |
| 16A | Water | | 0.4 | -0.2/- | -0.2/- |
| 16B | Water | 2% Chloride | 0.6 | 0.5/83 | 0.4/67 |
| 17 | Water | 2.5% Carbonate | 1.1 | -/100+ | -/- |
| 18 | Water | 5% Carbonate | 1.8 | -/100 | -/- |
| 19 | 3% Ammonia | 2% Carbonate | 1.6 | 1.7/100 | 1.2/75 |
| 19A | 3% Ammonia | | 0.6 | -0.3/- | -0.3/- |
| 19B | 3% Ammonia | 2% Chloride | 2 | 1.7/85 | 1.36/65 |

Examples 16-19 and particularly Example 12, when compared with Comparative Examples 16A and 16B,

and Example 19, when compared with Comparative Examples 19A and 19B, demonstrate the improved retention properties of carbonate quats over conventional chloride quats, particularly in the absence of metal stabilizers.

Indirect Synthesis of Carboxylate Quat

Example 20 - Didecyldimethylammonium propionate

180 grams (0.4 mole) of 80% didecyldimethylammonium chloride in 20% ethanol water (144 grams of DDAC), 180 ml of absolute denatured ethanol (denatured with methanol/isopropanol), and 32 grams (0.49 mole) of 85% potassium hydroxide pellets (27 grams of KOH) were mixed in a flask that was purged with nitrogen and equipped with a heating mantle and a magnetic stirrer. The mixture was stirred and heated at 60-70°C for three hours. The mixture was then allowed to cool to room temperature and finally cooled to 5°C.

Potassium chloride precipitated, and the precipitate was collected on a vacuum filter. The solid was washed with cold ethanol and subsequently was dried, yielding 31 grams (calculated yield 29.6 grams) of dry potassium chloride.

The ethanolic solution of the hydroxy quat containing about 0.09 mole of unreacted KOH, was stirred while 50 grams of carbon dioxide (from sublimed carbon dioxide) were bubbled over one half hour. The resultant mixture was then filtered to remove 7.2 grams of potassium carbonate (6.2 grams calculated), and the filtrate was concentrated to yield an orange/brown liquid with 80-85% carbonate quat (0.4 mole of carbonate quat) and less than 0.1% chloride for a product with 98 to 99% exchanged quat purity.

The cold product, after filtration, was placed in a closed flask equipped with a condenser, addition funnel, and a tube connected to a water displacement type gas measuring device. An equivalent (0.4 mole, 29.6 grams), of propionic acid was added to the carbonate quat over five minutes. Immediate gas evolution was noted, and 5.75 liters of gas were collected over 15 minutes. The solvent was removed on a rotary evaporator after the carbon dioxide evolution ceased, and yielded a yellow/orange liquid.

Quat analysis revealed that the product contained 85% active quat with 0.09% free chloride and 99% exchange.

Example 21 - Didecyldimethylammonium acetate

The procedure of Example 1 is followed, substituting 0.4 mole of acetic acid for the propionic acid.

Example 22 - Didecyldimethylammonium 2-ethylhexanoate

The procedure of Example 20 is followed, substituting 0.4 mole of 2-ethylhexanoic acid for the propionic acid.

The product is cloudy.

Example 23 - Didecyldimethylammonium gluconate

The procedure of Example 20 is followed, substituting 0.4 mole of gluconic acid for the propionic acid.

The product is water soluble.

Example 24 - Didecyldimethylammonium octanoate

The procedure of Example 20 is followed, substituting 0.4 mole of octanoic acid for the propionic acid.

Example 25 - Didecyldimethylammonium mixed coconut fatty acid carboxylate

The procedure of Example 20 is followed, substituting 0.4 mole of mixed coconut fatty acid for the propionic acid.

Example 26 - Didecyldimethylammonium laurate

The procedure of Example 20 is followed, substituting 0.4 mole of lauric acid for the propionic acid.

The product is a waxy solid.

Comparative Example 27 - Octyldecyldimethylammonium propionate

The procedure of Example 20 was followed, substituting 0.4 mole of 80% octyldecyldimethylammonium chloride for the didecyldimethylammonium chloride to yield octyldecyldimethylammonium propionate.

Comparative Example 28 - Octyldecyldimethylammonium acetate

The procedure of Example 21 was followed, substituting 0.4 mole of 80% octyldecyldimethylammonium chloride for the didecyldimethylammonium chloride to yield octyldecyldimethylammonium acetate.

Comparative Example 29 - Isononyldecyldimethylammonium 2-ethylhexanoate

The procedure of Example 22 was followed, substituting 0.4 mole of 80% isononyldecyldimethylammonium chloride for the didecyldimethylammonium chloride to yield isononyldecyldimethylammonium 2-ethyl-hexanoate.

Comparative Example 30 - Isononyldecyldimethylammonium gluconate

The procedure of Example 23 was followed, substituting 0.4 mole of 80% isononyldecyldimethylammonium chloride for the didecyldimethylammonium chloride to yield isononyldecyldimethylammonium gluconate.

Comparative Example 31 - Benzyldodecyldimethylammonium gluconate

The procedure of Example 23 was followed, substituting 0.4 mole of 80% benzyldodecyldimethylammonium chloride for the didecyldimethylammonium chloride to yield benzyldodecyldimethylammonium gluconate.

Comparative Example 32 - Benzyldodecyldimethylammonium octanoate

The procedure of Example 24 was followed, substituting 0.4 mole of 80% benzyldodecyldimethylammonium chloride for the didecyldimethylammonium chloride to yield benzyldodecyldimethylammonium octanoate.

Comparative Example 33 - A mixture of benzyldodecyl-, benzyltetradecyl-, and benzylhexadecyldimethylammonium mixed coconut fatty acid carboxylate

The procedure of Example 25 was followed, substituting 0.4 mole of 80% of a mixture of benzyldodecyl-, benzyltetradecyl-, and benzylhexadecyldimethylammonium mixed fatty acid benzyldodecyldimethylammonium chloride for the didecyldimethylammonium chloride to yield a mixture of benzyldodecyl-, benzyltetradecyl-, and benzylhexadecyldimethylammonium mixed coconut fatty acid carboxylate.

Comparative Example 34 - A mixture of benzyldodecyl-, benzyltetradecyl-, and benzylhexadecyldimethylammonium laurate

The procedure of Example 26 was followed, substituting 0.4 mole of 80% of a mixture of benzyldodecyl-, benzyltetradecyl-, and benzylhexadecyldimethylammonium chloride for the didecyldimethylammonium chloride to yield a mixture of benzyldodecyl-, benzyltetradecyl-, and benzylhexadecyldimethylammonium laurate.

Direct Synthesis of Carboxylate Quat

Example 35 - Didecyldimethylammonium acetate

180 grams (0.4 mole) of 80% didecyldimethylammonium chloride in 20% ethanol water (144 grams of DDAC), 180 ml of anhydrous ethanol, and a stoichiometric excess, 47 grams (0.48 mole), of anhydrous potassium acetate was mixed in a flask that was purged with nitrogen and equipped with a heating mantle, a magnetic stirrer, and a condenser. The mixture was stirred and heated at 60-70°C for two hours. The insoluble potassium acetate crystals slowly dissolved and a finer solid (KCl) separated. The mixture was then cooled to 0°C and vacuum filtered. The solid washed with cold ethanol to remove 30.7 grams of potassium chloride (theoretical 29.6 grams). The solution was concentrated, cooled, and filtered to remove 6.5 grams of potassium acetate (theoretical 29.6 grams).

Additional fine crystals of potassium acetate settled out on standing. By assay, the light yellow liquid product was determined to be 80% quat with 100% exchange.

Example 36 - Didecyldimethylammonium gluconate

0.0221 mole of sodium gluconate and 0.0221 mole of 80% didecyldimethylammonium chloride in water were mixed in a flask. The mixture was heated and held until evolution of carbon dioxide gas ceased.
The resultant quat was analyzed, and conversion was determined to be less than 20%.

Example 37 - Didecyldimethylammonium 2-ethylhexanoate

0.0221 mole of sodium 2-ethylhexanoate and 0.0221 mole of 80% didecyldimethylammonium chloride in water were mixed in a flask. The mixture was heated and held until evolution of carbon dioxide gas ceased.
The resultant quat was analyzed, and conversion was determined to be 77%.

Example 38 - Didecyldimethylammonium laurate

0.4 mole of sodium laurate and 0.4 mole of 80% didecyldimethylammonium chloride in water were mixed in a flask. The mixture was heated to 60°C and held for 1 hour.
The resultant quat was analyzed, and conversion was determined to be 90%

Example 39 - Didecyldimethylammonium propionate

0.0221 mole of sodium propionate and 0.0221 mole of 80% didecyldimethylammonium chloride in 8 grams of propionic acid were mixed in a flask. The mixture was heated to 60°C-80°C and held for 2 hours.
The resultant quat was analyzed, and conversion was determined to be 90%

Example 40 - Didecyldimethylammonium propionate

0.4 mole of potassium propionate and 0.4 mole of 80% didecyldimethylammonium chloride in solid form were mixed in a flask. The mixture was heated to 60°C - 80°C and held for 2 hours.
The resultant quat was analyzed, and conversion was determined to be 91%

Hydroxy Quat/Acid Synthesis

Comparative Example 41 - Didecyldimethylammonium propionate

180 grams (0.4 mole) of 80% didecyldimethylammonium chloride in 20% ethanol water (144 grams of DDAC), 180 ml of absolute denatured ethanol (denatured with methanol/isopropanol), and 26 grams (0.4 mole) of 85% potassium hydroxide pellets ( 22 grams of KOH) were mixed in a flask that was purged with nitrogen and equipped with a heating mantle and a magnetic stirrer. The mixture was stirred and heated at 60-70°C for three hours. The mixture was then allowed to cool to room temperature and finally cooled to 0°C for at least one hour.
Potassium chloride precipitated, and the precipitate was collected on a vacuum filter. The solid was washed with cold ethanol and subsequently was dried, yielding 30 grams of dry potassium chloride.
The hydroxy quat/ethanol solution was mixed with a stoichiometric amount of propionic acid to yield a yellow/orange liquid having a flash point of 106°F.

Comparative Example 42 - Didecyldimethylammonium borate

The procedure of Example 134 is followed substituting 0.4 mole of boric acid for the propionic acid.
The product is a liquid.

Comparative Examole 43 - Octyldecyldimethylammonium borate

The procedure of Example 42 was followed, substituting 0.4 mole of 80% octyldecyldimethylammonium chloride for the didecyldimethylammonium chloride to yield octyldecyldimethylammonium borate.

Comparative Examble 44 - Isononyldecyldimethylammonium borate

The procedure of Example 42 was followed, substituting 0.4 mole of 80% isononyldecyldimethylammonium chloride for the didecyldimethylammonium chloride to yield isononyldecyldimethylammonium borate.

Comparative Example 45 - Benzyldodecyldimethylammonium borate

The procedure of Example 42 was followed, substituting 0.4 mole of 80% benzyldodecyldimethylammonium chloride for the didecyldimethylammonium chloride to yield benzyldodecyldimethylammonium borate.

Comparative Example 46 - A mixture of benzyldodecyl-, benzyltetradecyl-, and benzylhexadecyldimethylammonium borate

The procedure of Example 42 was followed, substituting 0.4 mole of 80% of a mixture of benzyldodecyl-, benzyltetradecyl-, and benzylhexadecyldimethylammonium chloride for the didecyldimethylammonium chloride to yield a mixture of benzyldodecyl-, benzyltetradecyl-, and benzylhexadecyldimethylammonium borate.

Comparative Example 47 - Dihexadecyldimethylammonium borate

The procedure of Example 42 was followed, substituting 0.4 mole of 80% dihexadecyldimethylammonium chloride for the didecyldimethylammonium chloride to yield dihexadecyldimethylammonium borate.

Comparative Example 48 - Dodecyltrimethylammonium borate

The procedure of Example 42 was followed, substituting 0.4 mole of 80% dodecyltrimethylammonium chloride for the didecyldimethylammonium chloride to yield dodecyltrimethylammonium borate.

Treatment of Wood Substrate

Example 49 - Didecyldimethylammonium acetate

End grain pine wafers were weighed and then soaked with didecyldimethylammonium acetate in ethanol/water until a weight gain of 45% was observed.

The treated wafers were then placed in water and weighed periodically to determine resistance to leaching.

Results are illustrated in Figures 3A, 3B, and 3C.

Comparative Example 49A - Didecyldimethylammonium chloride

End grain pine wafers were weighed and then soaked with didecyldimethylammonium chloride in 20% ethanol/water until a weight gain of 35% was observed.

The treated wafers were then placed in water and weighed periodically to determine resistance to leaching.

Results are illustrated in Figures 3A, 3B and 3C.

Comparative Example 50 - Didecyldimethylammonium borate

End grain pine wafers were weighed and then soaked with didecyldimethylammonium borate in ethanol/water until a weight gain of 30% was observed.

The treated wafers were then placed in water and weighed periodically to determine resistance to leaching.

Results are illustrated in Figures 3A and 3B.

Example 51 - Didecyldimethylammonium methacrylate

End grain pine wafers were weighed and then soaked with didecyldimethylammonium methacrylate in ethanol/water until a weight gain of 30% was observed.

The treated wafers were then placed in water and weighed periodically to determine resistance to leaching.

Results are illustrated in Figures 1A and 1B.

Example 52 - Didecyldimethylammonium gluconate

End grain pine wafers were weighed and then soaked with didecyldimethylammonium gluconate in ethanol/water until a weight gain of 30% was observed.

The treated wafers were then placed in water and weighed periodically to determine resistance to leaching.

Results are illustrated in Figures 3A and 3B.

Example 53 - Didecyldimethylammonium propionate

End grain pine wafers were weighed and then soaked with didecyldimethylammonium propionate in ethanol/water until a weight gain of 30% was observed.
The treated wafers were then placed in water and weighed periodically to determine resistance to leaching.
Results are illustrated in Figures 3A and 3B.

Example 54 - Didecyldimethylammonium 2-ethylhexanoate

End grain pine wafers were weighed and then soaked with didecyldimethylammonium ethylhexanoate in ethanol/water until a weight gain of 35% was observed.
The treated wafers were then placed in water and weighed periodically to determine resistance to leaching.
Results are illustrated in Figure 3C.

Example 55 - Didecyldimethylammonium laurate

End grain pine wafers were weighed and then soaked with didecyldimethylammonium laurate in ethanol/water until a weight gain of 35% was observed.
The treated wafers were then placed in water and weighed periodically to determine resistance to leaching.
Results are illustrated in Figure 3C.

Example 56 - Didecyldimethylammonium decanoate

End grain pine wafers were weighed and then soaked with didecyldimethylammonium decanoate in ethanol/water until a weight gain of 30% was observed.
The treated wafers were then placed in water and weighed periodically to determine resistance to leaching.
Results are illustrated in Figure 3C.

Example 57 - Didecyldimethylammonium stearate

End grain pine wafers were weighed and then soaked with didecyldimethylammonium stearate in ethanol/water until a weight gain of 40% was observed.
The treated wafers were then placed in water and weighed periodically to determine resistance to leaching.
Results are illustrated in Figure 3C.

Example 58 - Didecyldimethylammonium stearate emulsion

End grain pine wafers were weighed and then soaked with didecyldimethylammonium stearate emulsion in water until a weight gain of 6% was observed.
The treated wafers were then placed in water and weighed periodically to determine resistance to leaching.
Results are illustrated in Figure 3C.

Example 59 - Didecyldimethylammonium octanoate

End grain pine wafers were weighed and then soaked with didecyldimethylammonium octanoate in water until a weight gain of 40% was observed.
The treated wafers were then placed in water and weighed periodically to determine resistance to leaching.
Results are illustrated in Figure 3C.
Figures 3A, 3B, and 3C illustrate that the carboxylate quats of the present invention resist leaching for extended periods of time, and better than the chloride quat.

Biocidal Activity

Example 60 - Didecyldimethylammonium acetate

Cultures of A. niger, G. trabeum, T. veride, and L. lepideus were inoculated with varying amounts of 75% of didecyldimethylammonium acetate in water. The concentrations of carboxylate quat at which no growth was observed and the highest concentration at which growth was not affected were averaged.

Results are illustrated in Table 6.

Comparative Example 60A - Didecyldimethylammonium chloride

The procedure of Example 60 was followed, substituting didecyldimethylammonium chloride for the didecyldimethylammonium acetate.

Results are illustrated in Table 6.

Comparative Example 60B - Didecyldimethylammonium chloride/iodopropargyl butylcarbamate

The procedure of Example 60 was followed, substituting a mixture of 4 parts of didecyldimethylammonium chloride and 1 part of iodopropargyl butylcarbamate for the didecyldimethylammonium acetate.

Results are illustrated in Table 6.

Example 61 - Didecyldimethylammonium 2-ethylhexanoate

The procedure of Example 60 was followed, substituting didecyldimethylammonium 2-ethylhexanoate for the didecyldimethyl-ammonium acetate.

Results are illustrated in Table 6.

Example 62 - Didecyldimethylammonium laurate

The procedure of Example 60 was followed, substituting didecyldimethylammonium laurate for the didecyldimethylammonium acetate.

Results are illustrated in Table 6.

Example 63 - Didecyldimethylammonium stearate

The procedure of Example 60 was followed, substituting didecyldimethylammonium stearate for the didecyldimethylammonium acetate.

Results are illustrated in Table 6.

Example 64 - Didecyldimethylammonium chloride (DDAC)/ polypropylene glycol monostearate (PGMS)/water

3 parts of didecyldimethylammonium chloride and 2.5 parts of PGMS are melted together and stirred while 94.5 parts of warm (40°C) water are added to yield a stable emulsion which is suitable for waterproofing and preserving wood.

Example 65 - DDAC/3% PGMS/Mineral Spirits

The method of Example 64 is followed substituting 3 parts of PGMS for the PGMS and 84 parts of mineral spirits for the water.

Example 66 - DDAC/6% PGMS/Mineral Spirits

The method of Example 65 is followed substituting 6 parts of PGMS for the PGMS and 91 parts of mineral spirits for the mineral spirits.

Example 67 - DDAC/8% PGMS/Mineral Spirits

The method of Example 65 is followed substituting 8 parts of PMGS for the PGMS and 89 parts of mineral spirits for the mineral spirits.

Example 68 - DDAC/12% PGMS/Mineral Spirits

The method of Example 65 is followed substituting 12 parts of PGMS for the PGMS and 85 parts of mineral spirits for the mineral spirits.

Example 69 - DDAC/9% ethylene glycol monostearate (EGMS)/Water

The method of Example 64 is followed substituting 9 parts of EGMS for the PGMS and 88 parts of water for the water.

Example 70 - DDAC/10% ethylene glycol distearate (EGDS)/Water

The method of Example 69 is followed substituting 10 parts of EGDS for the EGMS and 87 parts of water for the water.

Example 71 - DDAC/9% sorbitan tristearate (STS)/Water

The method of Example 64 is followed substituting 9 parts of STS for the PGMS and 88 parts of water for water.

Example 72 - DDAC/9% sorbitan monostearate (SMS)/Water

The method of Example 71 is followed substituting 9 parts of SMS for the STS.

Example 73 - DDAC/9% polyethylene glycol distearate (PEG 400-DS)/Water

The method of Example 71 is followed substituting 9 parts of PEG 400-DS for the SMS.

Example 74 - DDAC/9% PEG 400-DS/Mineral Spirits

The method of Example 73 is followed substituting 88 parts of mineral spirits for the water.

Example 75 - Didecyldimethylammonium hydroxide/PGMS/water

The method of Example 64 is followed substituting 3 parts of didecyldimethylammonium hydroxide for the didecyldimethylammonium chloride.

Example 76 - Didecyldimethylammonium carbonate/2.5% PGMS/water

The method of Example 64 is followed substituting 3 parts of didecyldimethylammonium carbonate for the didecyldimethylammonium chloride.

Example 77 - Didecyldimethylammonium carbonate/2.5% glycerol monolaureate (GML)/Water

The method of Example 76 is followed substituting 2.5 parts of GML for the PGMS.

Example 78 - Didecyldimethylammonium carbonate/2.5% glycerol monostearate (GMS)/Water

The method of Example 77 is followed substituting 2.5 parts of GMS for the GML.

Example 79 - Didecyldimethylammonium acetate/PGMS/water

The method of Example 64 is followed substituting 3 parts of didecyldimethylammonium acetate D for the didecyldimethylammonium chloride.

Example 80 - Didecyldimethylammonium mixed coconut fatty acid carboxylate/PGMS/water

The method of Example 64 is followed substituting 5 parts of didecyldimethylammonium mixed coconut fatty acid carboxylate prepared by the method of Procedure G for the didecyldimethylammonium chloride, 5 parts of PGMS for the PGMS, and 90 parts of water for the water.

Example 81 - Didecyldimethylammonium chloride/PGMS/water

End grain pine wafers are weighed and then soaked with a waterproofer, wood preservative system prepared

according to the method of Example 1 until the samples are saturated with the treating mixture. The samples are then air dried to constant weight to determine the uptake of the waterproofer, wood preservative system.

The treated wafers are removed, dried to constant weight, and weighed periodically to determine resistance to leaching.

The dried treated wafers are soaked in water for 30 minutes to determine swelling. Swell is measured as the increase in length of the sample compared to an untreated control, and the swell index for each is calculated.

Results are illustrated in Table 7 and Figures 4A and 4B.

Comparative Example 81A - Didecyldimethylammonium chloride

The method of Example 81 is followed substituting didecyldimethylammonium chloride for the waterproofer, wood preservative system.

Results are illustrated in Table 7 and Figures 4A and 4B.

Example 82 - DDAC/3% PGMS/Mineral Spirits

The method of Example 81 is followed substituting a waterproofer, wood preservative system prepared according to the method of Example 65 for the waterproofer, wood preservative system.

Results are illustrated in Table 7.

Example 83 - DDAC/6% PGMS/Mineral Spirits

The method of Example 81 is followed substituting a waterproofer, wood preservative system prepared according to the method of Example 66 for the waterproofer, wood preservative system.

Results are illustrated in Table 7.

Example 84 - DDAC/8% PGMS/Mineral Spirits

The method of Example 81 is followed substituting a waterproofer, wood preservative system prepared according to the method of Example 67 for the waterproofer, wood preservative system.

Results are illustrated in Table 7.

Example 85 - DDAC/12% PGMS/Mineral Spirits

The method of Example 81 is followed substituting a waterproofer, wood preservative system prepared according to the method of Example 68 for the waterproofer, wood preservative system.

Results are illustrated in Table 7.

Example 86 - DDAC/9% EGMS/Water

The method of Example 81 is followed substituting a waterproofer, wood preservative system prepared according to the method of Example 69 for the waterproofer, wood preservative system.

Results are illustrated in Table 7.

Example 87 - DDAC/10% EGDS/Water

The method of Example 81 is followed substituting a waterproofer, wood preservative system prepared according to the method of Example 70 for the waterproofer, wood preservative system.

Results are illustrated in Table 7.

Example 88 - DDAC/9% STS/Water

The method of Example 81 is followed substituting a waterproofer, wood preservative system prepared according to the method of Example 71 for the waterproofer, wood preservative system.

Results are illustrated in Table 7.

Example 89 - DDAC/9% SMS/Water

The method of Example 81 is followed substituting a waterproofer, wood preservative system prepared according to the method of Example 72 for the waterproofer, wood preservative system.
Results are illustrated in Table 7.

Example 90 - DDAC/9% PEG 400-DS/Water

The method of Example 81 is followed substituting a waterproofer, wood preservative system prepared according to the method of Example 73 for the waterproofer, wood preservative system.
Results are illustrated in Table 7.

Example 91 - DDAC/9% PEG 400-DS/Mineral Spirits

The method of Example 81 is followed substituting a waterproofer, wood preservative system prepared according to the method of Example 74 for the waterproofer, wood preservative system.
Results are illustrated in Table 1.

Example 92 - Didecyldimethylammonium hydroxide/PGMS/water

The method of Example 81 is followed substituting a waterproofer, wood preservative system prepared according to the method of Example 75 for the waterproofer, wood preservative system.
Results are illustrated in Table 7 and Figures 4A and 4B.

Comparative Example 92A - Didecyldimethylammonium hydroxide

The method of Comparative Example 81A is followed substituting didecyldimethylammonium hydroxide for the didecyldimethylammonium chloride.
Results are illustrated in Table 7 and Figures 4A and 4B.

Example 93 - Didecyldimethylammonium carbonate/2.5% PGMS/water

The method of Example 81 is followed, substituting a waterproofer, wood preservative system prepared according to the method of Example 76 for the waterproofer, wood preservative system.
Results are illustrated in Table 7 and Figures 4A and 4B.

Comparative Example 93A - Didecyldimethylammonium carbonate

The method of Comparative Example 81A is followed substituting didecyldimethylammonium carbonate for the didecyldimethylammonium chloride to yield a clear solution.
Results are illustrated in Table 7 and Figures 4A and 4B.

Example 94 - Didecyldimethylammonium carbonate/2.5% GML/Water

The method of Example 93 is followed substituting 2.5 parts of GML for the PGMS.
Results are illustrated in Table 7.

Example 96 - Didecyldimethylammonium carbonate/2.5% GMS/Water

The method of Example 81 is followed substituting 2.5 parts of GMS for the PGMS.
Results are illustrated in Table 7.

Examgle 96 - Didecyldimethylammonium acetate/PGMS/water

The method of Example 81 is followed, substituting a waterproofer, wood preservative system prepared according to the method of Example 16, for the waterproofer, wood preservative system.
Results are illustrated in Table 7 and Figures 4A and 4B.

Comparative Example 96A - Didecyldimethylammonium acetate

The method of Comparative Example 81A is followed substituting didecyldimethylammonium acetate for the didecyldimethylammonium chloride.
Results are illustrated in Figures 4A and 4B.

Example 97 - Didecyldimethylammonium mixed coconut fatty acid carboxylate/PGMS/water

The method of Example 81 is followed substituting a waterproofer, wood preservation system prepared according to the method of Example 80 for the waterproofer, wood preservative system to yield an emulsion.
Results are illustrated in Table 7.

Comparative Example 97A - Didecyldimethylammonium mixed coconut fatty acid carboxylate

The method of Comparative Example 81A is followed substituting 5 parts of didecyldimethylammonium mixed coconut fatty acid carboxylate and 95 parts of water for the waterproofer, wood preservative system.
Results are illustrated in Table 7.

Example 98 - PGMS

The method of Example 81 is followed substituting a solution of 8 parts of PGMS and 92 parts of water for the waterproofer, wood preservative system.
Results are illustrated in Table 7.

Comparative Example 98A - Mineral spirits

The method of Example 81 is followed substituting a commercially available wax based biocide/mineral spirit based solution (Woodtreat MB™ - KopCoat, Inc.)for the waterproofer, wood preservative system.
Results are illustrated in Table 7.
Table 7 illustrates the enhanced properties of waterproofer, wood preservative systems of the present invention.

## Claims

**1.** A preserved wood substrate obtained by treating a wood substrate with a wood preservative system comprising (a) a biocidal effective amount of at least one di-($C_8$-$C_{12}$ alkyl) quaternary ammonium hydroxide and (b) a solvent, wherein said wood preservative system is metal-free.

**2.** A preserved wood substrate as defined in claim 1, wherein said di-($C_8$-$C_{12}$ alkyl) quaternary ammonium hydroxide is didecyldimethylammonium hydroxide.

**3.** A preserved wood substrate as defined in claim 1, wherein said solvent is an aqueous solvent.

**4.** A preserved wood substrate as defined in claim 1, wherein said wood preservative system comprises from 0.1 to 5 parts by weight of di-($C_8$-$C_{12}$ alkyl) quaternary ammonium hydroxide and from 95 to 99.9 parts by weight of solvent, based upon 100 parts by weight of di-($C_8$-$C_{12}$ alkyl) quaternary ammonium hydroxide and solvent combined.

**5.** A wood preservative composition comprising

(a) at least one di-($C_8$-$C_{12}$ alkyl) quaternary ammonium carbonate having the formula

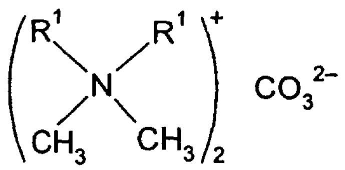

wherein $R^1$ is a $C_8$-$C_{12}$ alkyl group; and

(b) (1) at least one di-($C_8$-$C_{12}$ alkyl) quaternary ammonium bicarbonate having the formula

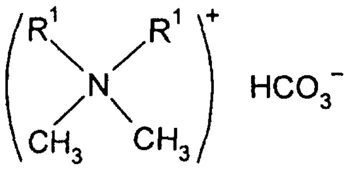

wherein $R^1$ is the same or different $C_8$-$C_{12}$ alkyl group as in (a); or

(2) at least one di-($C_8$-$C_{12}$ alkyl) quaternary ammonium metal carbonate having the formula

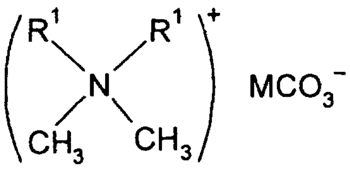

wherein $R^1$ is the same or different $C_8$-$C_{12}$ alkyl group as in (a) or (1) and M is a non-coupler metal, or

(3) a combination of (1) and (2);

said composition being metal coupler-free.

**6.** A wood preservative system comprising

(A) a biocidal effective amount of at least one di-($C_8$-$C_{12}$ alkyl) quaternary ammonium carbonate having the formula

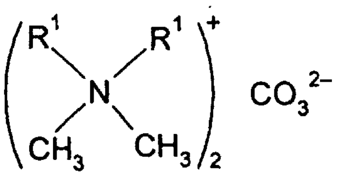

wherein $R^1$ is a $C_8$-$C_{12}$ alkyl group and

(B) a solvent;

wherein said system is metal coupler free.

**7.** A wood preservative system as defined in claim 6 comprising from 0.1 to 5 parts by weight of di-($C_8$-$C_{12}$ alkyl) quaternary ammonium carbonate and from 95 to 99.9 parts by weight of solvent, based upon 100 parts by weight of di-($C_8$-$C_{12}$ alkyl) quaternary ammonium carbonate and solvent combined.

**8.** A method for preserving a wood substrate comprising treating said wood substrate with a wood preservative system

as defined in claim 6.

**9.** A method for preserving a wood substrate comprising treating said wood substrate with a wood preservative composition as defined in claim 5.

**10.** Use of a wood preservative system comprising (a) a biocidal effective amount of at least one di-($C_8$-$C_{12}$ alkyl) quaternary ammonium hydroxide and (b) a solvent, wherein said wood preservative system is metal-free, for preserving a wood substrate.

**Patentansprüche**

**1.** Geschütztes Holzsubstrat, erhalten durch Behandlung eines Holzsubstrats mit einem Holzschutzsystem, welches (a) eine biozid wirksame Menge wenigstens eines quartären Di-($C_{8-12}$-alkyl)ammoniumhydroxids und (b) ein Lösungsmittel umfasst, worin das Holzschutzsystem metallfrei ist.

**2.** Geschütztes Holzsubstrat nach Anspruch 1, worin das quartäre Di-($C_{8-12}$-alkyl)-ammoniumhydroxid Didecyldimethylammoniumhydroxid ist.

**3.** Geschütztes Holzsubstrat nach Anspruch 1, worin das Lösungsmittel ein wässriges Lösungsmittel ist.

**4.** Geschütztes Holzsubstrat nach Anspruch 1, worin das Holzschutzsystem 0,1 bis 5 Gewichtsteile quartäres Di-($C_{8-12}$-alkyl)ammoniumhydroxid und 95 bis 99,9 Gewichtsteile Lösungsmittel, bezogen auf zusammen 100 Gewichtsteile quartäres Di-($C_{8-12}$-alkyl)-ammoniumhydroxid und Lösungsmittel, umfasst.

**5.** Holzschutzzusammensetzung, die

(a) wenigstens ein quartäres Di-($C_8$-$C_{12}$-alkyl)ammoniumcarbonat der Formel

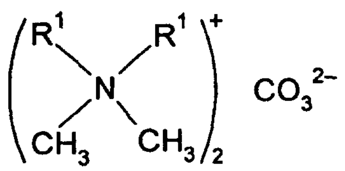

worin $R^1$ eine $C_8$-$C_{12}$-Alkylgruppe ist, und

(b)(1) wenigstens ein quartäres Di-($C_8$-$C_{12}$-alkyl)ammoniumbicarbonat der Formel

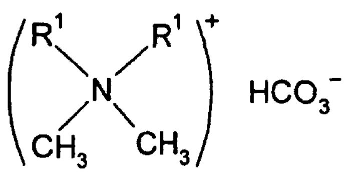

worin $R^1$ die gleiche oder eine andere $C_8$-$C_{12}$-Alkylgruppe wie in (a) ist, oder
(2) wenigstens ein quartäres Di-($C_8$-$C_{12}$-alkylammonium-metallcarbonat der Formel

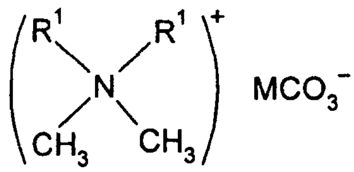

worin $R^1$ die gleiche oder eine andere $C_8$-$C_{12}$-Alkylgruppe wie in (a) oder (1) ist, oder
(3) eine Kombination von (1) und (2),
umfasst und frei von Metallkuppler ist.

**6.** Holzschutzsystem, welches

(A) eine biozid wirksame Menge wenigstens eines quartären Di-($C_8$-$C_{12}$-alkyl)-ammoniumcarbonats der Formel

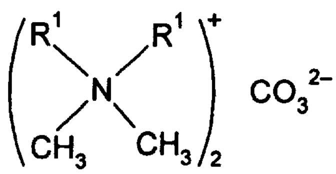

worin $R^1$ eine $C_8$-$C_{12}$-Alkylgruppe ist, und
(B) ein Lösungsmittel
umfasst und frei von Metallkuppler ist.

**7.** Holzschutzsystem nach Anspruch 6, umfassend 0,1 bis 5 Gewichtsteile quartäres Di-($C_8$-$C_{12}$-alkyl)ammoniumcarbonat und 95 bis 99,9 Gewichtsteile Lösungsmittel, bezogen auf zusammen 100 Gewichtsteile quartäres Di-($C_8$-$C_{12}$-alkyl)ammoniumcarbonat und Lösungsmittel.

**8.** Verfahren zum Schützen eines Holzsubstrats, welches die Behandlung des Holzsubstrats mit einem Holzschutzsystem gemäss Anspruch 6 umfasst.

**9.** Verfahren zum Schützen eines Holzsubstrats, welches die Behandlung des Holzsubstrats mit einer Holzschutzzusammensetzung gemäss Anspruch 5 umfasst.

**10.** Verwendung eines Holzschutzsystems, welches (a) eine biozid wirksame Menge wenigstens eines quartären Di-($C_8$-$C_{12}$-alkyl)ammoniumhydroxids und (b) ein Lösungsmittel umfasst und metallfrei ist, zum Schutz eines Holzsubstrats.

**Revendications**

**1.** Support bois préservé, obtenu par le traitement d'un support bois avec système de préservation du bois comprenant (a) une quantité efficace d'un biocide constitué d'au moins un hydroxyde d'ammonium quaternaire de dialkyl ($C_8$-$C_{12}$) et (b) un solvant, dans lequel ledit système de préservation du bois ne contient aucun métal.

**2.** Support bois préservé selon la revendication 1, dans lequel ledit hydroxyde d'ammonium quaternaire de di-alkyl ($C_8$-$C_{12}$) est l'hydroxyde de didécyldiméthylammonium.

**3.** Support bois préservé selon la revendication 1, dans lequel ledit solvant est un solvant aqueux.

**4.** Support bois préservé selon la revendication 1, comprenant une proportion pondérale de 0,1 à 5 parties d'hydroxyde d'ammonium quaternaire de dialkyl($C_8$-$C_{12}$) et une proportion pondérale de 95 à 99,9 parties de solvant pour

une combinaison constituée de 100 parties pondérales d'hydroxyde d'ammonium quaternaire de dialkyl($C_8$-$C_{12}$) et de solvant.

5. Composition d'un préservateur du bois, comprenant

(a) au moins un carbonate d'ammonium quaternaire de dialkyl($C_8$-$C_{12}$) ayant la formule

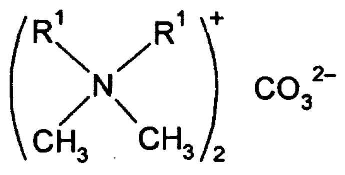

dans laquelle $R^1$ est un groupe alkyl($C_8$-$C_{12}$) ; et
(b) (1) au moins un bicarbonate d'ammonium quaternaire de dialkyl($C_8$-$C_{12}$) ayant la formule

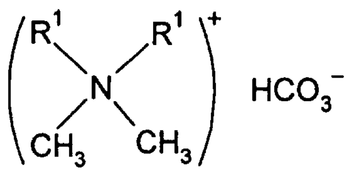

dans laquelle $R^1$ est un groupe alkyl($C_8$-$C_{12}$) identique à celui figurant dans (a) ou différent de celui-ci ; ou
(2) au moins un carbonate métallique d'ammonium quaternaire de dialkyl($C_8$-$C_{12}$) ayant la formule

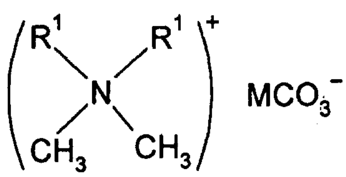

dans laquelle $R^1$ est un groupe alkyl($C_8$-$C_{12}$) identique à celui figurant dans (a) ou dans (1) ou différent de celui figurant dans (a) ou dans (1) et M est un métal non copulant ; ou
(3) combinaison de (1) et de (2),
ladite composition ne contenant pas de copulant métallique.

6. Système de préservation du bois comprenant (A) une quantité efficace d'un biocide constitué d'au moins un carbonate d'ammonium quaternaire de dialkyl($C_8$-$C_{12}$) ayant la formule

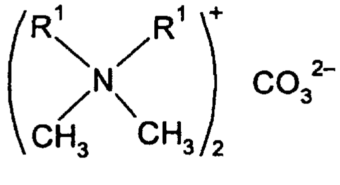

dans laquelle $R^1$ est un groupe alkyl($C_8$-$C_{12}$) et (B) un solvant ; dans lequel ledit système ne contient pas de copulant métallique.

7. Système de préservation du bois selon la revendication 6, comprenant une proportion pondérale de 0,1 à 5 parties de carbonate d'ammonium quaternaire de dialkyl($C_8$-$C_{12}$) et une proportion pondérale de 95 à 99,9 parties de solvant pour une combinaison constituée de 100 parties pondérales de carbonate d'ammonium quaternaire de

dialkyl($C_8$-$C_{12}$) et de solvant.

**8.** Méthode de préservation d'un support bois comprenant le traitement dudit support bois avec un système de préservation du bois selon la revendication 6.

**9.** Méthode de préservation d'un support bois comprenant le traitement dudit support bois avec un préservateur de bois selon la revendication 5.

**10.** Utilisation d'un système de préservation du bois comprenant (a) une quantité efficace d'un' biocide constitué d'au moins un hydroxyde d'ammonium quaternaire de dialkyl($C_8$-$C_{12}$) présente dans le support bois et (b) un solvant, dans lequel ledit système de préservation du bois ne contient aucun métal, pour la préservation d'un support bois.

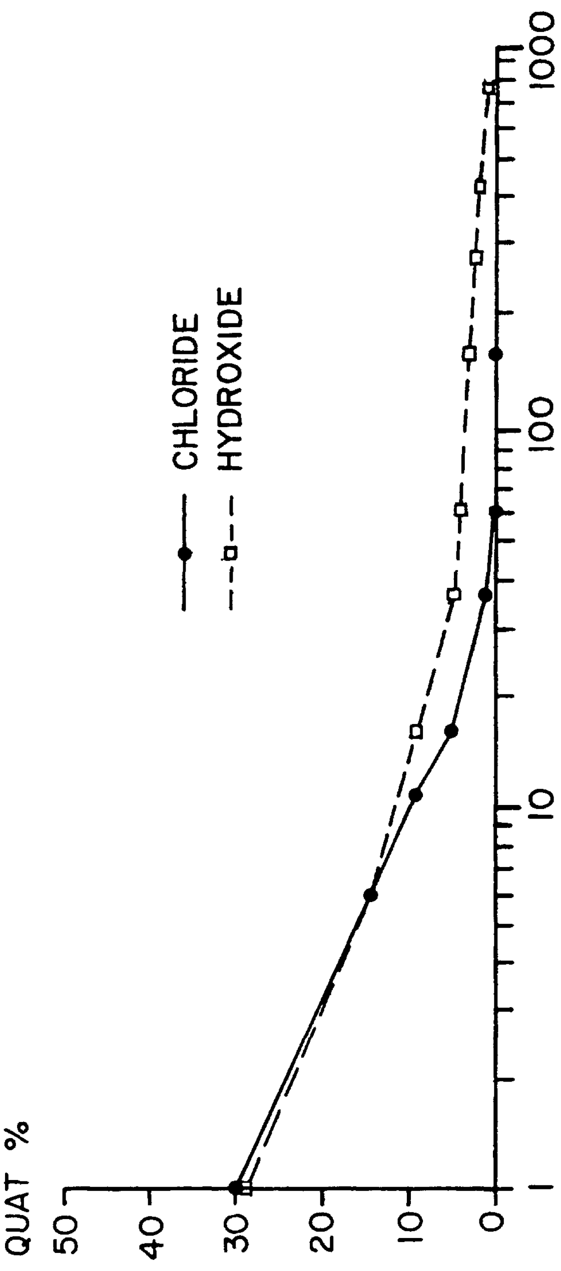
QUAT %
50
40
30
20
10
0
1
10
100
1000
TIME OF LEACHING (HOURS)
CHLORIDE
HYDROXIDE

FIG. 1A

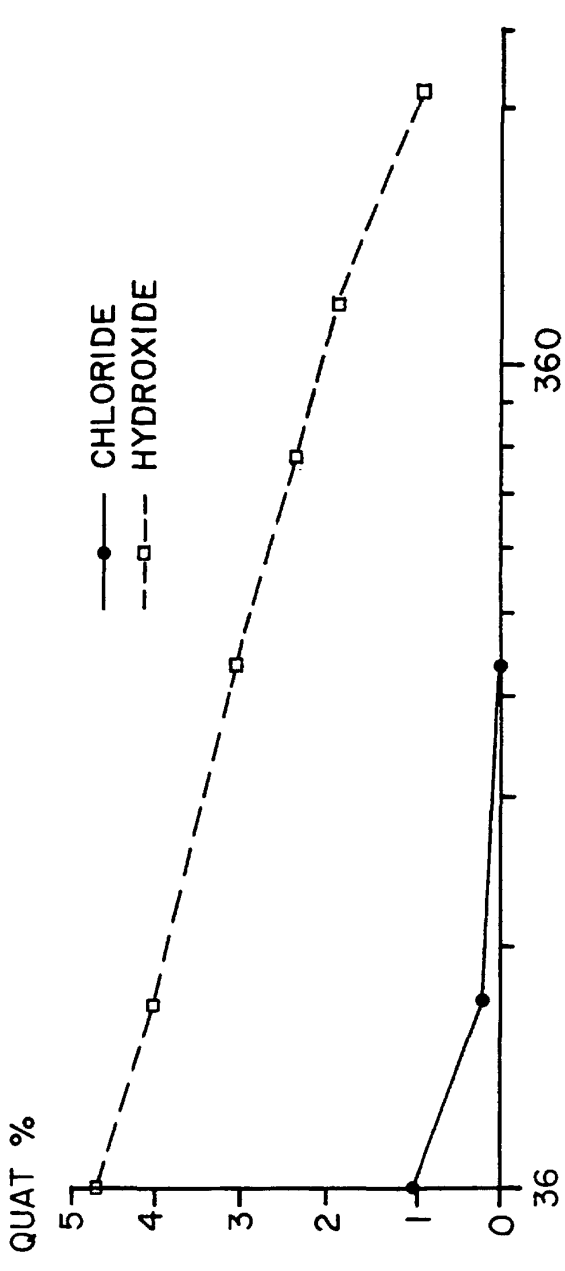
QUAT %
5
4
3
2
1
0
36
360
TIME OF LEACHING (HOURS)
CHLORIDE
HYDROXIDE

FIG. 1B

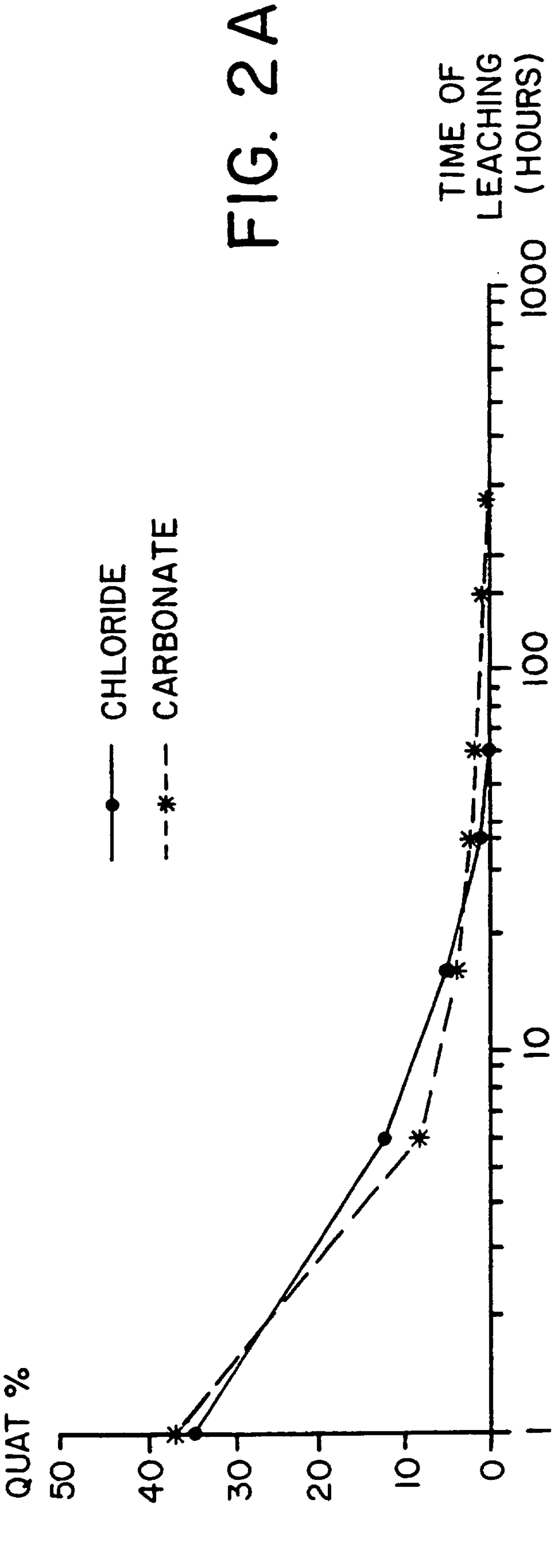
QUAT %
50
40
30
20
10
0
1
10
100
1000
TIME OF LEACHING (HOURS)
CHLORIDE
CARBONATE

FIG. 2A

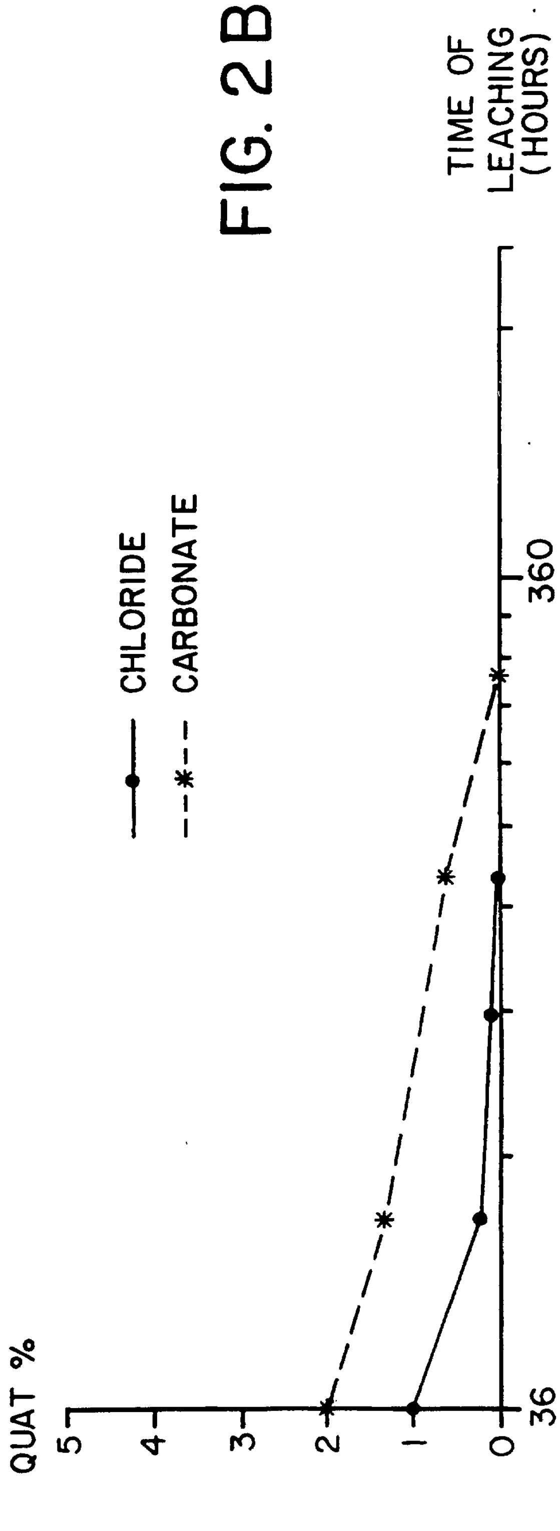
QUAT %
5
4
3
2
1
0
36
360
TIME OF LEACHING (HOURS)
CHLORIDE
CARBONATE

FIG. 2B

FIG. 3A

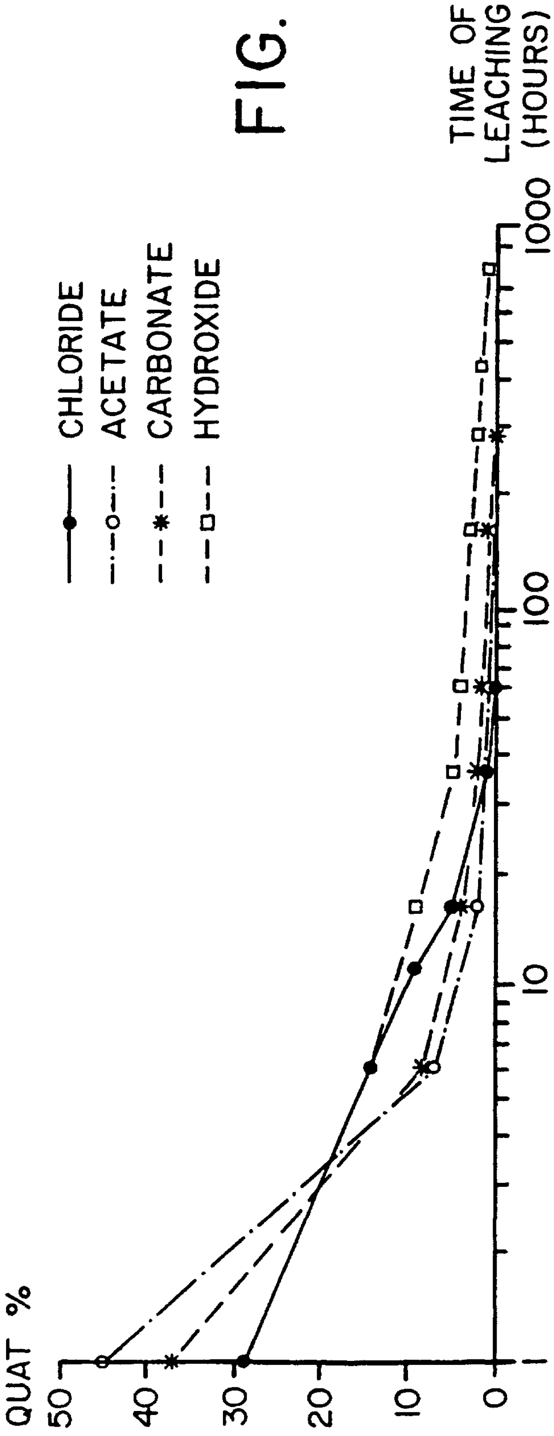
QUAT %
50
40
30
20
10
0
10
100
1000
TIME OF LEACHING (HOURS)
CHLORIDE
ACETATE
CARBONATE
HYDROXIDE

FIG. 3B

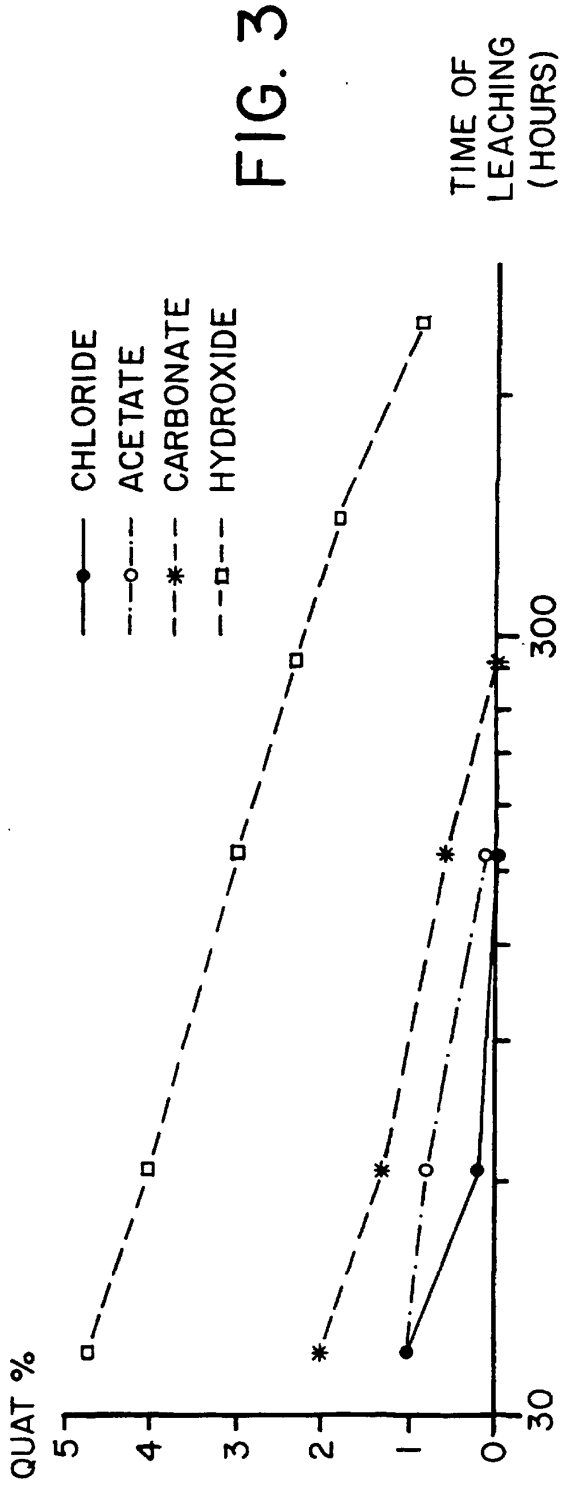
QUAT %
5
4
3
2
1
0
30
300
TIME OF LEACHING (HOURS)
CHLORIDE
ACETATE
CARBONATE
HYDROXIDE

FIG. 3C

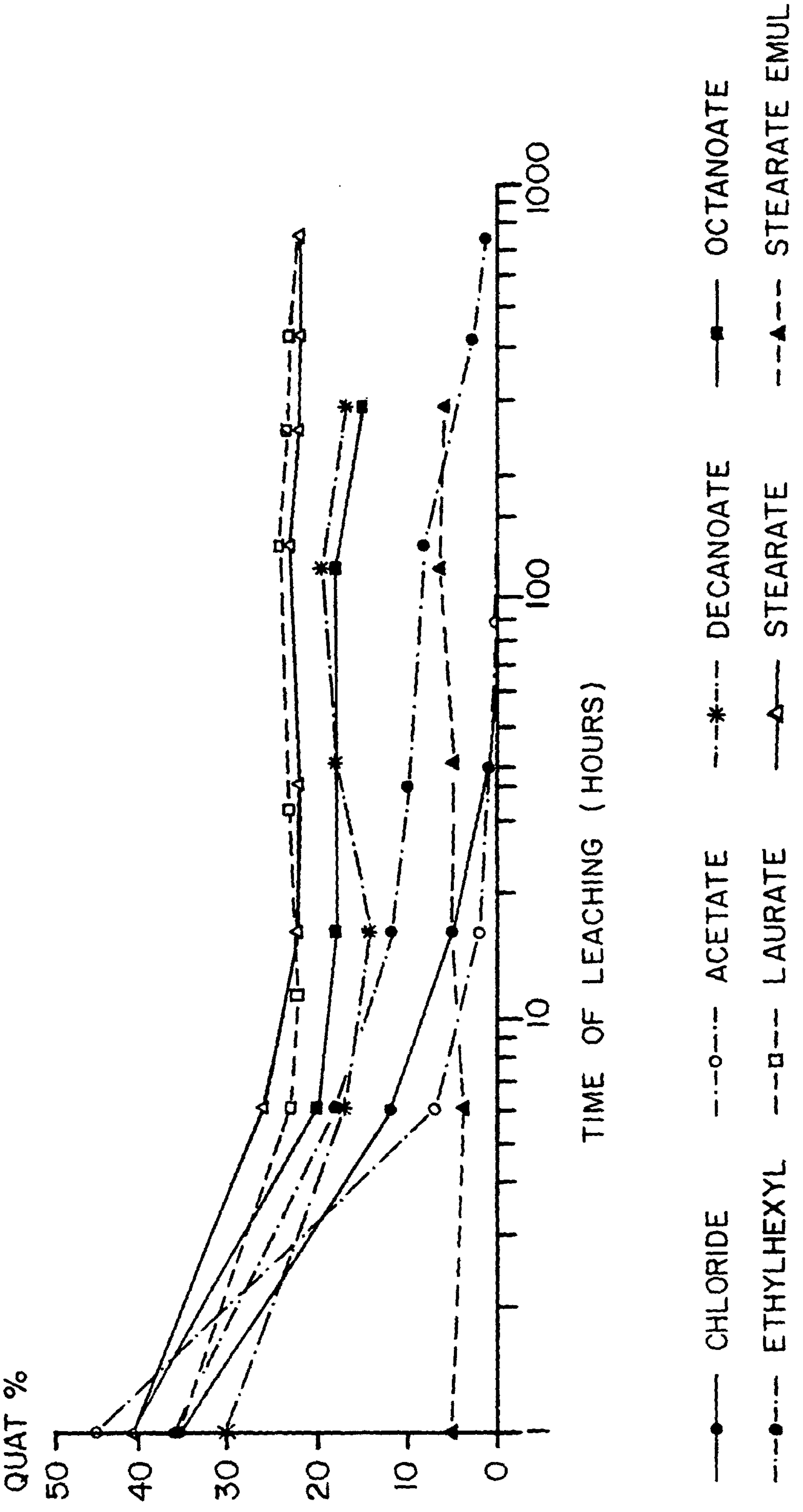
QUAT %
50
40
30
20
10
0
10
100
1000
TIME OF LEACHING (HOURS)
CHLORIDE
ACETATE
DECANOATE
OCTANOATE
ETHYLHEXYL
LAURATE
STEARATE
STEARATE EMUL

FIG. 4A

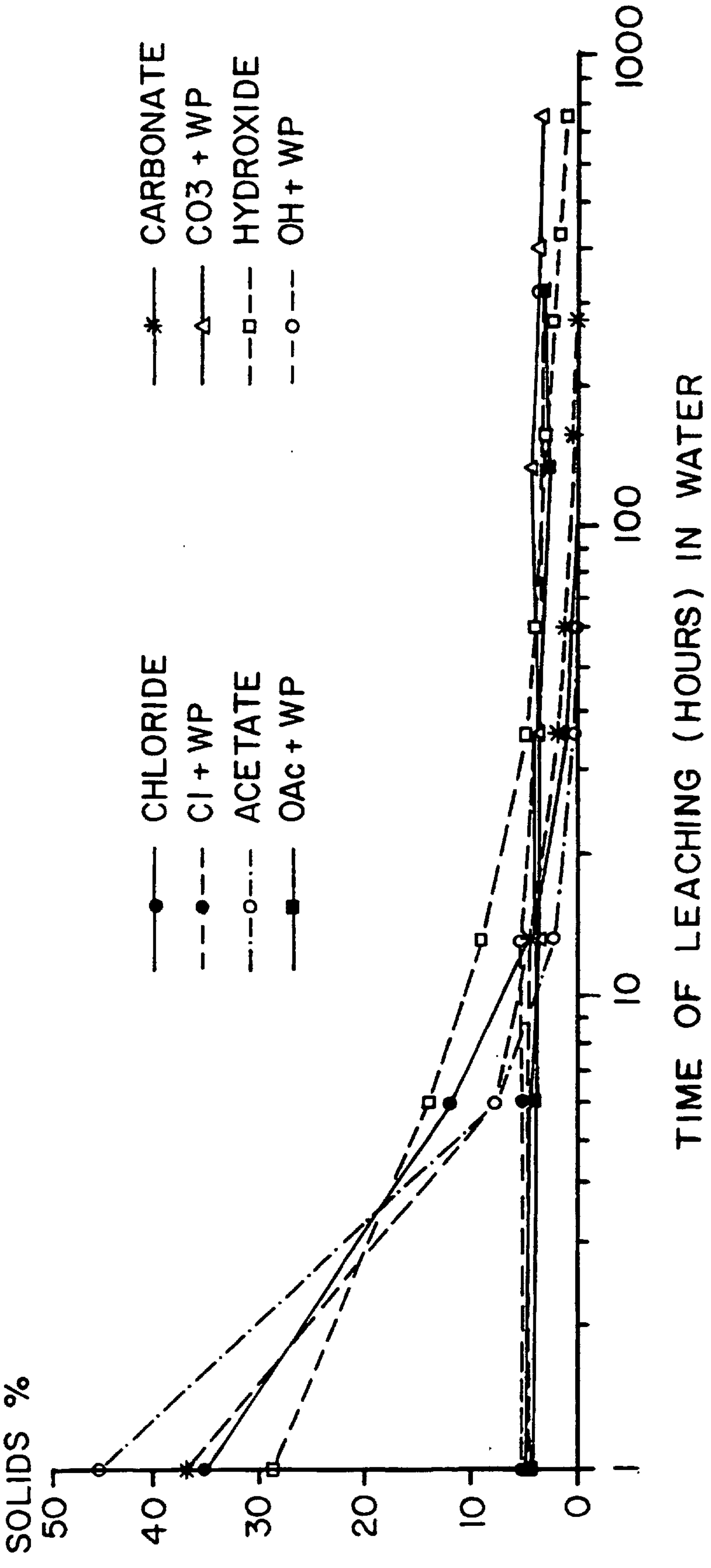
SOLIDS %
50
40
30
20
10
0
1
10
100
1000
TIME OF LEACHING (HOURS) IN WATER
CHLORIDE
Cl + WP
ACETATE
OAc + WP
CARBONATE
CO3 + WP
HYDROXIDE
OH + WP

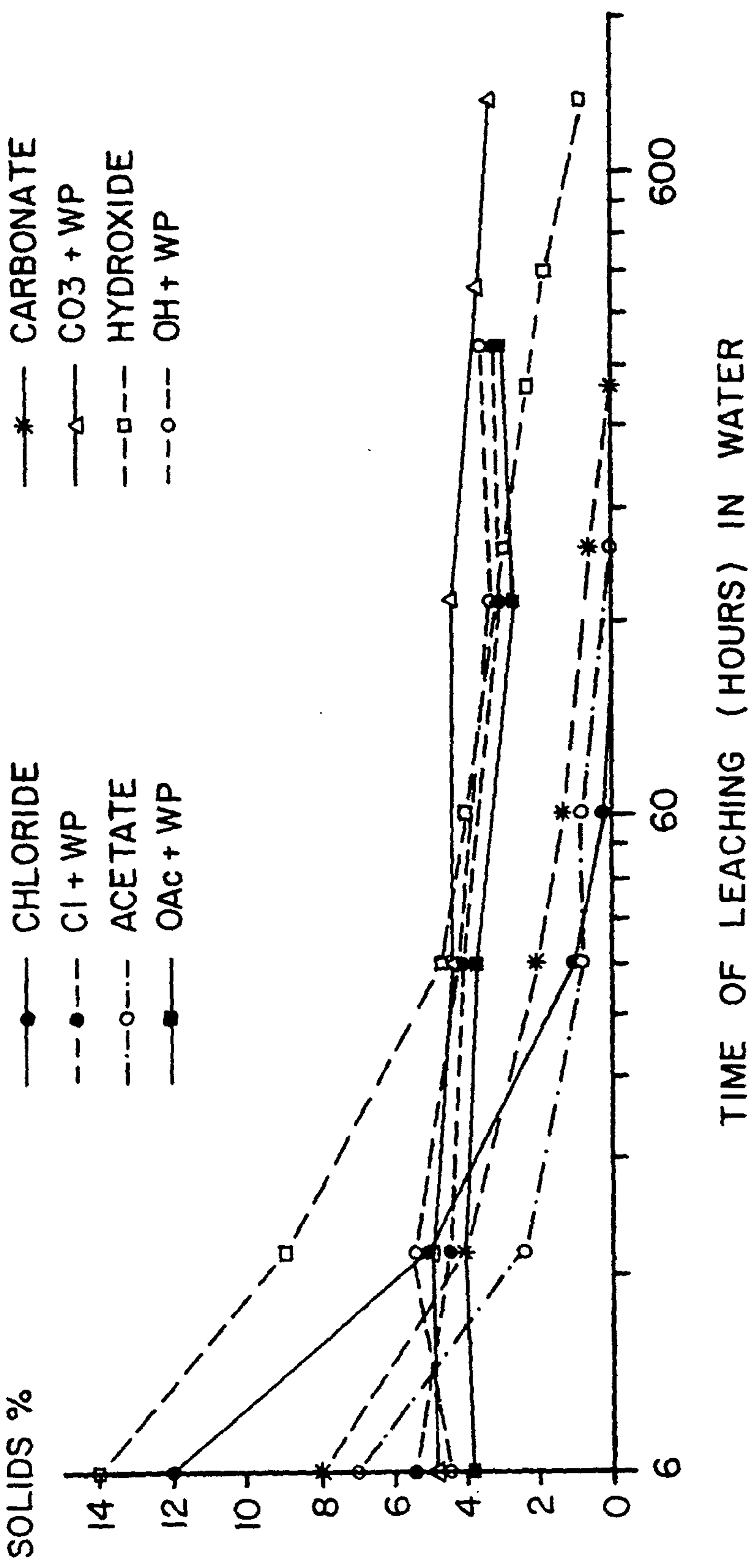
SOLIDS %
14
12
10
8
6
4
2
0
6
60
600
TIME OF LEACHING (HOURS) IN WATER
CHLORIDE
Cl + WP
ACETATE
OAc + WP
CARBONATE
CO3 + WP
HYDROXIDE
OH + WP

FIG. 4B